(19) Europäisches Patentamt
European Patent Office
Office européen des brevets

(11) **EP 3 615 147 B1**

(12) **EUROPÄISCHE PATENTSCHRIFT**

(45) Veröffentlichungstag und Bekanntmachung des Hinweises auf die Patenterteilung:
**11.09.2024 Patentblatt 2024/37**

(21) Anmeldenummer: **17720478.1**

(22) Anmeldetag: **28.04.2017**

(51) Internationale Patentklassifikation (IPC):
**A61Q 5/00** (2006.01)   **A61Q 11/00** (2006.01)
**A61Q 19/00** (2006.01)   **A61K 8/97** (2017.01)
**A61K 36/28** (2006.01)   **A61K 9/00** (2006.01)
**A61K 47/10** (2017.01)   **A61K 47/36** (2006.01)
**A61K 9/107** (2006.01)   **A61P 17/00** (2006.01)

(52) Gemeinsame Patentklassifikation (CPC):
**A61Q 5/00; A61K 8/97; A61K 9/0014; A61K 9/107;**
**A61K 36/28; A61K 47/10; A61K 47/36;**
**A61P 17/00; A61Q 11/00; A61Q 19/00;**
A61K 2236/00

(86) Internationale Anmeldenummer:
**PCT/EP2017/060175**

(87) Internationale Veröffentlichungsnummer:
**WO 2018/196993 (01.11.2018 Gazette 2018/44)**

(54) **SCHAFGARBE FRISCHPFLANZENPRESSSAFT KONZENTRAT, HERSTELLUNG UND VERWENDUNG**

YARROW FRESH-PLANT PRESSED JUICE CONCENTRATE, PRODUCTION, AND USE

CONCENTRÉ DE JUS PRESSÉ DE PLANTE FRAÎCHE D'ACHILLÉE, PRODUCTION ET UTILISATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**04.03.2020 Patentblatt 2020/10**

(73) Patentinhaber: **Symrise AG**
**37603 Holzminden (DE)**

(72) Erfinder:
• **HERRMANN, Martina**
  **31789 Hameln (DE)**
• **GAEBLER, Sandra**
  **37671 Höxter (DE)**
• **PESARO, Manuel**
  **37688 Beverungen (DE)**
• **STUHLMANN, Dominik**
  **37603 Holzminden (DE)**
• **GARBE, Lisa**
  **37603 Holzminden (DE)**
• **MEYER, Julia**
  **32694 Dörentrup (DE)**

(74) Vertreter: **Eisenführ Speiser**
**Patentanwälte Rechtsanwälte PartGmbB**
**Postfach 31 02 60**
**80102 München (DE)**

(56) Entgegenhaltungen:
**WO-A1-97/33596      WO-A2-97/42963**
**JP-A- 2004 250 354**

• **BENEDEK ET AL: "Achillea millefolium L. s.l. - Is the anti-inflammatory activity mediated by protease inhibition?", JOURNAL OF ETHNOPHARMACOLOGY, ELSEVIER IRELAND LTD, IE, vol. 113, no. 2, 15 August 2007 (2007-08-15), pages 312 - 317, XP022200242, ISSN: 0378-8741, DOI: 10.1016/J.JEP.2007.06.014**
• **TESSAROLLO ET AL: "Fitodefensivos em plantas medicinais", 1 January 2013 (2013-01-01), pages 180 - 187, XP055378403, Retrieved from the Internet <URL:http://www.scielo.br/pdf/rbpm/v15n2/03.pdf> [retrieved on 20170602]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Bemerkungen:

Die Akte enthält technische Angaben, die nach dem Eingang der Anmeldung eingereicht wurden und die nicht in dieser Patentschrift enthalten sind.

Bemerkungen:

Die Akte enthält technische Angaben, die nach dem Eingang der Anmeldung eingereicht wurden und die nicht in dieser Patentschrift enthalten sind.

# EP 3 615 147 B1

## Beschreibung

Die vorliegende Erfindung betrifft ein festes Schafgarbe Frischpflanzenpresssaft Konzentrat, das langfristig gelagert werden kann, ohne dabei auf thermische Behandlung, Konservierungsmittel oder andere Zusatzstoffe zurückgreifen zu müssen. Weiterhin betrifft die Erfindung ein schonendes Verfahren zur Herstellung eines derartigen Schafgarbe Frischpflanzenpresssaft Konzentrats. Das Schafgarbe Frischpflanzenpresssaft Konzentrat wird in kosmetischen Zusammensetzungen, pharmazeutischen Zusammensetzungen, Nahrungsmittelzusammensetzungen oder Nahrungsergänzungsmitteln eingesetzt.

Die vorliegende Erfindung betrifft außerdem neue Gesichtspunkte der Verwendung von Schafgarbe. Die Erfindung stellt die Verwendung von Schafgarbe zur Stimulierung der Expression von Hitzeschockproteinen und/oder antimikrobiellen Peptiden in Hautzellen bereit. Ferner wird die Verwendung von Schafgarbe zur Erhöhung der Thermotoleranz der Haut beschrieben.

Die Schafgarbe (*Achillea millefollium*) ist eine traditionelle Heilpflanze. Typische Inhaltsstoffe umfassen beispielsweise ätherische Öle, Sesquiterpene, phenolische Verbindungen, wie beispielsweise Flavonoide, Phenolcarbonsäuren, und Betaine. Beispielhaft Phenolcarbonsäuren umfassen Chlorogensäure und verschiedene Dicaffeoylchinasäuren.

Die Schafgarbe wird in Form von Extrakten eingesetzt. Für die Herstellung flüssiger Extrakte werden beispielsweise frische (nicht getrocknete) Pflanzen genutzt, d.h. in der Regel muss das pflanzliche Ausgangsmaterial in der Nähe der Produktionsfläche kultiviert oder gesammelt werden. Die erntereifen Pflanzen werden geerntet und müssen zeitnah (in der Regel im Laufe eines Tages) gepresst und abgefüllt werden, um Verderb und mikrobielle Beeinträchtigung auszuschließen.

Derartig erhaltener Schafgarbenkraut-Presssaft kann beispielsweise bei leichten krampfartigen Beschwerden im Magen-Darm einschließlich Bähungen und Appetitlosigkeit verwendet werden. Außerdem findet Schafgarbe in Hautcremes aufgrund ihrer positiven Eigenschaften bei der Wundheilung Anwendung.

WO 97/33596 A1 offenbart Verfahren zum Herstellen eines lagerfähigen Präparats aus Frischpflanzen und Frischpflanzenpräparaten. WO 97/33596 A1 beschreibt Schafgarbe als Heilpflanze.

Schafgarbe-Extrakte werden in der Kosmetik für topische Applikationen bereits vielfach eingesetzt. Dabei handelt es sich um Extrakte, die aus der getrockneten Droge hergestellt werden. Die Trocknung bietet zeitliche Unabhängigkeit von dem saisonalen Erntezeitraum sowie Schutz vor dem möglichen Verderb. Trockenes Pflanzenmaterial lässt sich einfacher und kostengünstiger lagern und transportieren. Jedoch kommt es sowohl bei der Trocknung des Pflanzenmaterials als auch bei der Herstellung des Extrakts (Extraktion ggf. unter Wärmezufuhr und üblicherweise anschließende destillative Entfernung des Extraktionsmittels unter Erhitzen zur Aufkonzentrierung) zur teilweisen Veränderungen des Inhaltsstoffspektrums der Pflanze.

Die industrielle Produktion von Schafkraut Frischpflanzenpresssäften erfolgt im Allgemeinen durch Ernte der zu pressenden Pflanzen bzw. Pflanzenteile, Reinigung, Zerkleinern, Wasserdampfbehandlung zum Inaktivieren von Pflanzenenzymen und Aufschließen des Pflanzenmaterials, Pressen unter Druck, Filtrieren oder Zentrifugieren, Ultrahochkurzzeiterhitzung zum Sterilisieren und aseptisches Abfüllen (Deutsche Apothekerzeitung 2011, S. 93 ff.). Derartige Frischpflanzenpresssäfte enthalten für gewöhnlich 2-6 Gew.-% Trockenextrakt bzw. Trockensaft. Es handelt sich um ein schonendes Verfahren, bei dem nur kurzfristig höhere Temperaturen zum Einsatz gelangen. Weiterhin sind vergleichsweise wenige Arbeitsschritte erforderlich. Dadurch soll einen Schutz für empfindliche, biologisch aktive Inhaltsstoffe, wie z.B. Antioxidantien, gewährleistet werden.

Von Nachteil ist, dass der erhaltene Frischpflanzenpresssaft nur im geschlossenen Behälter gut lagerbar ist, da sonst Gefahr mikrobieller Kontamination besteht. In Folge ist der Frischpflanzenpresssaft, v.a. für den Endverbraucher, bei der Handhabung problematisch. Diese rührt daher, dass entweder der Komplettverbrauch notwendig ist, oder der Zusatz unerwünschter Konservierungsmittel zu dem Frischpflanzenpresssaft zum Schutz vor Verderb notwendig ist.

Daher besteht eine Aufgabe der vorliegenden Erfindung in der Bereitstellung langfristig lagerbarer Schafgarbenextrakte und eines Verfahrens zur Herstellung derartiger Extrakte. Eine weitere Aufgabe besteht in der Bereitstellung eines Schafgarbenextrakts, das ohne die Verwendung von Konservierungsmitteln oder anderer Zusatzstoffe gelagert werden kann. Das Profil der Inhaltsstoffe sollen weder durch das Herstellungsverfahren noch durch die Lagerung beeinträchtigt werden. Weiterhin soll ein wässriges Schafgarbenextrakt bereitgestellt werden, das im Vergleich zu den im Stand der Technik bekannten Anwendungsmöglichkeiten weitere, zusätzlich vorteilhafte Anwendungen bietet. So sind im Stand der Technik Wirkstoffe zur Stimulierung von Hitzeschockproteinen und/oder kutanen AMPs beschrieben. Dennoch besteht ein Bedarf an neuen natürlichen, gut verträglichen, einfach in kosmetischen Formulierungen einbringbaren HSP und/oder AMP Stimulatoren.

Die Erfindung betrifft daher ein festes Schafgarbe Frischpflanzenpresssaft Konzentrat, umfassend oder bestehend aus:

10-50 Gew.-% Schafgarbe Frischpflanzenpresssaft Trockenrückstand,

0-10 Gew.-% Wasser, und

50-90 Gew.-% Trägerstoff(e),

bezogen auf 100 Gew.-% des Schafgarbe Frischpflanzenpresssaft Konzentrats,

wobei der bzw. die Trägerstoff(e) ein fester Trägerstoff, ausgewählt aus der Gruppe bestehend aus Maltodextrin, Dextrin und Cyclodextrin, ist bzw. sind,

wobei das Scharfgarbe Frischpflanzenpresssaft Konzentrat eine oder mehrere Dicaffeoylchinasäure(n) enthält, wobei eine oder mehrere oder alle Dicaffeoylchinasäure(n) ausgewählt ist/sind aus der Gruppe bestehend aus 3,4-Dicaffeoylchinasäure, 3,5-Dicaffeoylchinasäure und 4,5-Dicaffeoylchinasäure.

Weiterhin betrifft die Erfindung ein Verfahren zur Herstellung eines erfindungsgemäßen Schafgarben Frischpflanzenpresssaft Konzentrats, umfassend die Schritte oder bestehend aus den Schritten:

Bereitstellen eines Schafgarbe Frischpflanzenpresssafts, und

Konzentrieren des Schafgarbe Frischpflanzenpresssafts.

Zudem betrifft die vorliegende Erfindung eine kosmetische Zusammensetzung umfassend ein erfindungsgemäßes Schafgarbe Frischpflanzenpresssaft Konzentrat oder Schafgarbe Frischpflanzenpresssaft.
Die Erfindung stellt die kosmetische, nicht-therapeutische Verwendung von Schafgarbe zur Stimulierung der Expression von Hitzeschockproteinen in Hautzellen bereit.
Die Erfindung stellt ferner die kosmetische, nicht therapeutische Verwendung von Schafgarbe zur Erhöhung der Thermotoleranz der Haut bereit.
Darüber hinaus betrifft die vorliegende Erfindung eine Pharmazeutische Zusammensetzung, Nahrungsmittelzusammensetzung oder Nahrungsergänzungsmittel, umfassend ein erfindungsgemäßes Schafgarbe Frischpflanzenpresssaft Konzentrat und die pharmazeutische Zusammensetzung zur Verwendung als Medikament.
Überraschenderweise wurde gefunden, dass Konzentrate aus Schafgarbe Frischpflanzenpresssaft Trockenrückstand langfristig haltbar sind. Dies ist sogar unter Weglassung von Konservierungsstoffen oder entsprechender Behandlungsverfahren bei erhöhter Temperatur, bspw. Pasteurisieren, zum Haltbarmachen der Fall. Das erfindungsgemäße Verfahren ermöglicht die schonende (d.h. keine längere Exposition bei höheren Temperaturen) Verfahren zum Bewahren des Inhaltsstoffprofils. Hierzu können insbesondere die Umkehrosmose, Gefriertrocknung, Sprühtrocknung oder eine Kombination dieser Konzentrierungsverfahren verwendet werden.
Durch den Einsatz eines oder mehrerer dieser Verfahren können nachteilige Vorgehensweisen zur Konzentrierung von Frischpflanzenpresssaft, wie die destillative Entfernung des Wassers, vermieden werden. Die destillative Entfernung des Wassers erfordert längere Prozessdauer bei erhöhten Temperaturen (70-80°C), was in der Regel zu Veränderungen beim Inhaltsstoffprofil führt. Thermisch sensible Inhaltsstoffe können sich dabei verändern bzw. abgebaut werden.
Es wurde weiterhin gefunden, dass die Schafgarbe im Allgemeinen, insbesondere die erfindungsgemäßen Konzentrate und Schafgarbe Frischpflanzenpresssäfte, für kosmetische Zusammensetzungen geeignet sind. So reguliert Schafgarbe, insbesondere Schafgarbe Frischpflanzenpresssaft, manche Hizeschockproteins (HSPs) auf Genebene hoch. So wird HSP27 nicht aber HSP70 auf Genebene hochreguliert. Weiterhin konnte überraschenderweise gefunden werden, dass Schafgarbe, insbesondere Schafgarbe Frischpflanzenpresssaft, antimikrobielle Proteine und Peptide (AMPs), wie beispielsweise beta-Defensin 1 und S100 Calciumbindeprotein A8, auf Genebene hochreguliert. Es ist bekannt, dass Hsp27 Thermotoleranz und Zytoprotektion bereitstellt und das Überleben von Zellen unter Stressbedingungen unterstützt. AMPs hingegen können als topisch angewendete Biozide Verwendung finden, insbesondere zur Behandlung multiresistenter Krankheitserreger und zur Behandlung von Haut- und Wundinfektionen. Weiterhin ist die Verwendung bei der Tumortherapie denkbar. Dies führt zu vorteilhaften Anwendungen von Schafgarbe v.a. im Rahmen von kosmetischen Zusammensetzungen und pharmazeutischen Zusammensetzungen, die vorzugsweise topisch appliziert werden.
Der Begriff "Schafgarbe" wie hierin verwendet betrifft *Achillea millefolium* Kraut bzw. Schafgarbenkraut und ebenso die Mitglieder der *Achillea millefolium-Gruppe* gemäß Hagers Handbuch der pharmazeutischen Praxis, Springer Verlag. Die Schafgarbe kann als unbehandelte Pflanze oder in verschieden Zubereitungen, bspw. Schafgarbe Frischpflanzenpresssaft oder Schafgarbe Frischpflanzenpresssaft Konzentrat, vorliegen.
Der "Schafgarbe Frischpflanzenpresssaft" und das "Schafgarbe Frischpflanzenpresssaft Konzentrat" sind durch das Vorhandensein eines relativ hohen Gehalts an Polyphenolen v.a. Phenolcarbonsäuren darunter die Gruppe der Monocaffeoylchinasäuren (beinhaltend Chlorogensäure [3-O-Caffeoylchinasäure, CAS 327-97-9], Neochlorgensäure [5-O-Caffeoylchinasäure CAS 906-33-2] und Cryptochlorogensäure [4- O-Caffeoylchinasäure, CAS 905-99-7]) sowie die Gruppe der Dicaffeoylchinasäuren (beinhaltend 1,5- Di-O-Caffeoylchinasäure [Cynarin, CAS 30964-13-7], 3,4- Di-O-Caffeoylchinasäure [Isochlorogensäure B, CAS 14534-61-3], 3,5-Di-O-Caffeoylchinasäure [Isochlorogensäure A, CAS 2450-53-5] und 4,5-Di-O-Dicaffeoylchinasäure [Isochlorogensäure C, CAS 57378-72-0]) gekennzeichnet. Polyphenole sind bekannt für ihre sehr gute anti-oxidative Wirksamkeit.

Schafgarbe Frischpflanzenpresssaft kann beispielsweise von der Firma Schoenenberger bezogen werden (Gebrauchsinformation: Naturreiner Heilpflanzensaft Schafgarbe zur Anwendung für Erwachsene und Jugendliche über 12 Jahren, Walther Schoenenberger Pflanzensaftwerk GmbH & Co. KG, Magstadt (Deutschland), Juli 2014). Der Schafgarbe Frischpflanzenpresssaft wird aus frischem, zur Blütezeit geerntetem Schafgarbekraut hergestellt. Er wird zur oralen Anwendung als traditionelles pflanzliches Arzneimittel bei leichten krampfartigen Beschwerden im Magen-Darm-Bereich einschließlich Blähungen und bei Appetitlosigkeit auf den Markt gebracht. Der Schafgarbe Frischpflanzenpresssaft ist eine braune, klare bis leicht opaleszierende Flüssigkeit und enthält üblicherweise zwischen 94,5 - 96,5 Gew.-% Wasser und 3,5 - 5,5 Gew.-% Trockenrückstand.

Schafgarbe Frischpflanzenpresssaft Konzentrat kann beispielsweise ausgehend vom Schafgarbe Frischpflanzenpresssaft durch ein Konzentrierungsverfahren hergestellt werden. Beispielsweise wird Schafgarbe Frischpflanzenpresssaft Konzentrat aus dem Schafgarbe Frischpflanzenpresssaft der Firma Schoenenberger durch Konzentrieren hergestellt. Es ist jedoch klar, dass jeglicher Schafgarbe Extrakt, insbesondere Schafgarbe Frischpflanzenpresssaft, verwendet werden kann.

Der Begriff "Trockenrückstand" oder "Trockensubstanz" wird gemäß der Definition in der analytischen Chemie für den nach dem Trocknen verbleibenden Wasser- und/oder Lösungsmittelfreien Anteil eines Stoffes, beispielsweise eines Schafgarbe Frischpflanzenpresssafts oder Schafgarbe Frischpflanzenpresssaft Konzentrats, verwendet. Das Trocknen verläuft hierbei im Wesentlichen vollständig, d.h. ein verbleibender Wasserund/oder Lösungsmittelgehalt des analysierten Stoffen, beispielsweise eines Schafgarbe Frischpflanzenpresssafts oder Schafgarbe Frischpflanzenpresssaft Konzentrats, ist vorzugsweise $\leq 1$ Gew.-%, vorzugsweise $\leq 0.5$ Gew.-%, $\leq 0.1$ Gew.-%, mehr bevorzugt $\leq 0.01$ Gew.-%. Das Trocknen erfolgt hierbei vorzugsweise durch schonende Verfahren, bspw. durch eine geringfügig erhöhte Temperatur, wie bspw. $\leq 10°C$, $\leq 5°C$ oder $\leq 2°C$, als der Siedepunkt von Wasser, dem Lösungsmittel bzw. ggf. dem Lösungsmittelgemisch. Alternative Möglichkeiten zum Trocken bestehen bspw. im Gefriertrocknen, Trocknen unter partiellem Vakuum, Verwendung von Trockenmitteln oder einer Kombination der vorstehend genannten Verfahren.

Der Begriff "Extrakt" wie hierin verwendetet betrifft eingedickte oder eingetrocknete Auszüge aus pflanzlichen Stoffen, insbesondere aus Schafgarbe. Mithin ist ein Extrakt eine Flüssigkeit. Schafgarbe Frischpflanzenpresssaft stellt einen derartigen Extrakt dar. Schafgarbe Frischpflanzenpresssaft enthält vorzugsweise $\leq 8$ Gew.-% Trockenrückstand, vorzugsweise $\leq 7,5$ Gew.-% Trockenrückstand, $\leq 7$ Gew. Trockenrückstand, $\leq 6,5$Gew.-% Trockenrückstand, $\leq 6$ Gew.-% Trockenrückstand oder $\leq 5,5$ Gew.-% Trockenrückstand. Mehr bevorzugt enthält Schafgarbe Frischpflanzenpresssaft 3,5 Gew.-% bis 5,5 Gew.-% Trockenrückstand.

Der Begriff "Konzentrat" wie hierin verwendet betrifft einen Feststoff oder eine Flüssigkeit mit einem relativ geringen Anteil an zusätzlichen Füllstoffen und/oder Lösungsmitteln. Schafgarbe Frischpflanzenpresssaft Konzentrat zeichnet sich somit gegenüber dem Schafgarbe Frischpflanzenpresssaft durch einen höheren Trockenrückstand aus. Schafgarbe Frischpflanzenpresssaft Konzentrat weist $\geq 10$ Gew.-% Trockenrückstand, vorzugsweise $\geq 15$ Gew.-% Trockenrückstand, $\geq 20$ Gew.-% Trockenrückstand, $\geq 25$ Gew.-% Trockenrückstand, $\geq 30$ Gew.-% Trockenrückstand, $\geq 40$ Gew.-% Trockenrückstand, $\geq 50$ Gew.-% Trockenrückstand, $\geq 60$ Gew.-% Trockenrückstand, $\geq 70$ Gew.-% Trockenrückstand, $\geq 80$ Gew.-% Trockenrückstand, oder $\geq 90$ Gew.-% Trockenrückstand auf. Das Konzentrat wird schonend erzeugt, d.h. dass im Vergleich zum Extrakt im Wesentlichen das gleiche Inhaltsspektrum an Bestandteilen entsprechend konzentriert (gemäß dem Verhältnis Trockenrückstand Konzentrat dividiert durch Trockenrückstand Extrakt) erhalten bleibt. Im Wesentlichen das gleiche Inhaltsspektrum an Bestandteilen betrifft individuelle Abweichungen der Bestandteile von $\pm 10$ Gew.-% im Konzentrat, vorzugsweise $\pm 5$ Gew.-% im Konzentrat, mehr bevorzugt $\pm 2$ Gew.-% im Konzentrat. Weist beispielsweise Schafgarbe Frischpflanzenpresssaft einen Trockenrückstand von 8 Gew.-% und Schafgarbe Frischpflanzenpresssaft Konzentrat einen Trockenrückstand von $\geq 10$ Gew.-% auf sind, so liegt die Konzentration der individuellen Bestandteile bzw. Inhaltsstoffe im Konzentrat mindestens um den Faktor 10 dividiert durch 8 erhöht vor.

Festes Schafgarbe Frischpflanzenpresssaft Konzentrat umfasst, bezogen auf 100 Gew.-% Schafgarbe Frischpflanzenpresssaft Konzentrat, 10-50 Gew.-% Schafgarbe Frischpflanzenpresssaft Trockenrückstand, 0-10 Gew.-% Wasser, und 50-90 Gew.-% Trägerstoff bezogen auf 100 Gew.-% des Schafgarbe Frischpflanzenpresssaft Konzentrats. Vorzugsweise wird festes Schafgarbe Frischpflanzenpresssaft Konzentrat direkt, d.h. ohne vorherige Konzentrierung, unter optionalem Zusatz der hierin genannten Trägerstoffe, hergestellt. Derartiges festes Schafgarbe Frischpflanzenpresssaft Konzentrat umfasst 10-50 Gew.-% Schafgarbe Frischpflanzenpresssaft Trockenrückstand, 0-10 Gew.- % Wasser, und 50-90 Gew.- % Trägerstoff.

Flüssiges Schafgarbe Frischpflanzenpresssaft Konzentrat wird vorzugsweise durch Umkehrosmose und anschließendes Zugeben eines flüssigen Trägerstoffs hergestellt und umfasst vorzugsweise 10-70 Gew.-% Schafgarbe Frischpflanzenpresssaft Trockenrückstand, 0-90 Gew.-% Wasser, 0-90 Gew.-% flüssigen Trägerstoff, sowie 0-5 Gew.-

% Konservierungsmittel und/oder Stabilisatoren. Der/die optionale(en) flüssige(n) Trägerstoff(e) ist bzw. sind wie nachstehend hierin aufgeführt und ist/sind oder umfasst/umfassen bspw. Glycerin, Propylenglycol, Butylenglycol, 1,3-Propandiol, 1,2-Pentandiol, 1,2-Hexandiol und wird/werden ebenso wie das optionale Konservierungsmittel und/oder der Stabilisator nach der Konzentrierung zugegeben.

Bevorzugt ist flüssiges Schafgarbe Frischpflanzenpresssaft Konzentrat umfassend 10-50 Gew.-% Schafgarbe Frischpflanzenpresssaft Trockenrückstand, 0-90 Gew.-% Wasser, 0-50 Gew.-% Trägerstoff, 0-3 Gew.-% Konservierungsmittel und/oder Stabilisatoren. Der Vorteil von festem oder flüssigem Schafgarbe Frischpflanzenpresssaft Konzentrat besteht in der einfachen und kostengünstige Herstellung, der guten Lagerbarkeit, der einfachen Handhabbarkeit und Dosierbarkeit, sowie, falls gewünscht, Standardisierbarkeit.

Die Dosierung von (flüssigem oder festem) Schafgarbe Frischpflanzenpresssaft Konzentrat in kosmetischen Zusammensetzungen, insbesondere in kosmetischen Zusammensetzung zur Reinigung und Pflege von Haut und Haar, beträgt 0.0001-10 Gew.-%, bevorzugt 0.001-5 Gew.-% und besonders bevorzugt 0.005-3 Gew.-%.

Vorzugsweise weisen kosmetische Zusammensetzungen 0.0001-10 Gew.-%, bevorzugt 0.001-5 Gew.-% und besonders bevorzugt 0.005-3 Gew.-% Schafgarbe Frischpflanzenpresssaft Konzentrat auf, wobei das Konzentrat mittels Gefriertrocknung, Sprühtrocknung oder Umkehrosmose erhalten werden kann. Diese kosmetischen Zusammensetzungen enthalten weiterhin eine der in Beispiel 5, Tabelle 12, Zusammensetzungen 1-11 genannten Inhaltsstoffkombinationen, wobei die individuelle Menge der jeweiligen Inhaltsstoffe neben dem Schafgarbe Frischpflanzenpresssaft Konzentrat frei gewählt werden kann. Es ist klar, dass die eingesetzten Duftstoffkombinationen nicht auf die Parfümöle PFO1 bzw. PFO2 begrenzt sind, sondern auch andere, dem Fachmann geläufige, Duftstoffe bzw. Duftstoffkombinationen verwendet werden können.

Alternativ kann eine Gelzahncreme vorliegen. Diese weist ebenso 0.0001-10 Gew.-%, bevorzugt 0.001-5 Gew.-% und besonders bevorzugt 0.005-3 Gew.-% Schafgarbe Frischpflanzenpresssaft Konzentrat auf, wobei das Konzentrat mittels Gefriertrocknung, Sprühtrocknung oder Umkehrosmose erhalten werden kann. Diese Zusammensetzung enthält weiterhin eine der in Beispiel 5, Tabelle 13 Zusammensetzungen I, II und III genannten Inhaltsstoffkombinationen, wobei die individuelle Menge der jeweiligen Inhaltsstoffe neben dem Schafgarbe Frischpflanzenpresssaft Konzentrat frei gewählt werden kann.

Alternativ kann eine gebrauchsfertige Mundspülung mit Fluorid vorliegen. Diese weist ebenso 0.0001-10 Gew.-%, bevorzugt 0.001-5 Gew.-% und besonders bevorzugt 0.005-3 Gew.-% Schafgarbe Frischpflanzenpresssaft Konzentrat auf, wobei das Konzentrat mittels Gefriertrocknung, Sprühtrocknung oder Umkehrosmose erhalten werden kann. Diese Zusammensetzung enthält weiterhin eine der in Beispiel 5, Tabelle 14, Zusammensetzungen I, II und III genannten Inhaltsstoffkombinationen, wobei die individuelle Menge der jeweiligen Inhaltsstoffe neben dem Schafgarbe Frischpflanzenpresssaft Konzentrat frei gewählt werden kann.

Alternativ besteht das erfindungsgemäße Schafgarbe Frischpflanzenpresssaft Konzentrat aus den genannten Inhaltstoffen. In diesem Fall sind keine weiteren Inhaltstoffe vorhanden. Somit besteht das erfindungsgemäße Schafgarbe Frischpflanzenpresssaft Konzentrat aus, bezogen auf 100 Gew.-% Schafgarbe Frischpflanzenpresssaft Konzentrat, 10-50 Gew.-% Schafgarbe Frischpflanzenpresssaft Trockenrückstand, 0-10 Gew.-% Wasser, und 50-90 Gew.-% Trägerstoff.

Der Zusatz von Trägerstoffen bzw. Trägermaterial erlaubt in gewissem Rahmen die Beeinflussung der Produkteigenschaften (z.B. Fließfähigkeit) und/oder Standardisierung. Die resultierenden Matrixpartikel bieten darüber hinaus auch einen gewissen Schutz für labile Inhaltsstoffe. Der Trägerstoff wird vorzugsweise derart gewählt, dass er im Rahmen der Konzentrierung zur Anwendung gelangt. Erfolgt beispielsweise die Konzentrierung mittels Sprühtrocknung kann als geeignetes Material ein fester Trägerstoff, insbesondere eines der nachstehend genannten Polysaccharide, verwendet werden. Dieses Polysaccharid kann wiederum in bspw. einer kosmetischen Zusammensetzung als Trägerstoff dienen, der der Zusammensetzung die gewünschten Eigenschaften verleiht. Mehr bevorzugt wird im Rahmen der Konzentrierung ein Trägerstoff verwendet, der in der gewünschten Darreichungsform, bspw. in einer kosmetischen Zusammensetzung, pharmazeutischen Zusammensetzung, Nahrungsmittelzusammensetzung oder in einem Nahrungsergänzungsmittel zur Verwendung gelangt.

Diese Trägerstoffe können fest oder flüssig sein (bei 25°C und 1013 mbar). Als Trägerstoffe können Einzelsubstanzen oder auch Substanzgemische eingesetzt werden.

Ein besonders bevorzugter fester Trägerstoff ist Maltodextrin.

Der Zersetzungsgrad der Stärke wird üblicherweise mit der Kennzahl "Dextrose-Equivalent" (DE) angegeben, welche die Grenzwerte 0 für ein langkettiges Glucosepolymer und 100 für die reine Glucose annehmen kann. Polysaccharide, insbesondere Maltodextrin, mit DE-Werten im Bereich 5 bis 20, vorzugsweise im Bereich 15 bis 20, sind wiederum besonders vorteilhaft.

Besonders bevorzugte Trägerstoffe sind Maltodextrine, wobei hier wiederum Maltodextrine mit DE-Werten im Bereich 5 bis 20 bevorzugt sind.

Die bevorzugten bzw. besonders bevorzugten Trägerstoffe weisen ferner den Vorteil auf, dass sie geschmacksneutral bzw. im Wesentlichen geschmacksneutral sind. Dadurch können erfindungsgemäße Konzentrate in vielen verschiedenen Produkttypen und Zubereitungen eingesetzt werden, da diese Vorprodukte das sonstige sensorische Profile, insbesondere das Aroma- und Geschmacksprofil - abgesehen von den unangenehmen, zu maskierenden Geschmackseindrücken - nicht oder nicht nennenswert beeinflussen.

Gemäß einer Ausführungsform umfasst das Schafgarbe Frischpflanzenpresssaft Konzentrat weiterhin Konservierungsmittel und/oder Stabilisatoren.

Jegliche Zahl von Konservierungsmitteln und/oder Stabilisatoren können verwendet werden.

Konservierungsmittel, die im Rahmen der vorliegenden Erfindung zum Einsatz gelangen, weisen im Regelfall mehrere, vorzugsweise alle, der nachstehend genannten Eigenschaften auf. Sie sind toxikologisch unbedenklich, gut hautverträglich, stabil, im Wesentlichen vollständig geruchsfrei und einfach und kostengünstig (d.h. aus einfach zugänglichen Edukten) herzustellen. Das Konservierungsmittel kann ferner antimikrobielle Eigenschaften aufweisen. Geeignete Konservierungsmittel können beispielsweise der WO 2006/045743 entnommen werden.

Beispiele geeigneter Konservierungsmittel umfassen Benzoesäure, die Ester und Salze von Benzoesäure, Sorbinsäure und seine Salze, Propionsäure und seine Salze, Salicylsäure und seine Salze, Dormaldehyd und Paraformaldehyd, 2-Hydroxybiphenylether und seine Salze, 2-Zinksulfidopyridin-N-oxid, anorganische Sulfite und Bisulfites, Natriumiodat, Chlorobutanol, 4-Ethylquecksilber(II)-5-amino-1,3-bis(2-hydroxybenzoesäure), die Ester und Salze davon, Dehydracetsäure, Ameisensäure, 1,6-Bis(4-amidino-2-bromophenoxy)-n-hexan und seine Salze, das Natriumsalz von Ethylquecksilber(II)-thiosalicylsäure, Phenylquecksilber und seine Salze, 10-Undecylensäure und seine Salze, 5-Amino-1,3-bis(2-ethylhexyl)-5- methylhexahydropyrimidin, 5-Bromo-5-nitro-1,3-dioxan, 2-Bromo-2-nitro-1,3-propandiol, 2,4-Dichlorobenzylalkohol, N-(4-chlorophenyl)-N'-(3,4-dichlorophenyl)harnstoff, 4-Chloro-m-cresol, 2,4,4'-Trichloro-2'-hydroxydiphenylether, 4-Chloro-3,5-dimethylphenol, 1,1'-Methylen-bis(3-(1-hydroxymethyl-2,4-dioximidazolidin-5-yl)harnstoff), Poly(hexamethylenbiguanid)hydrochlorid, 2-Phenoxyethanol, Hexamethylentetramin, 1(3-Chloroallyl)-3,5,7-triaza-1-azoniaadamantanchlorid, 1 (4-Chlorophenoxy)1(1H-imidazole-1 -yl)-3,3-dimethyl-2-butanon, 1,3-Bis(hydroxymethyl)-5,5-dimethyl-2,4-imidazolidindion, Benzylalkohol, Octopirox, 1,2-Dibromo-2,4-dicyanobutan, 2,2'-Methylen-bis(6-bromo-4-chlorophenol), Bromochlorophen, Gemisch aus 5-Chloro-2-methyl-3(2H)isothiazolinon und 2-Methyl-3(2H)isothiazolinon mit Magnesiumchlorid und Magnesiumnitrat, 2-Benzyl-4-chlorophenol, 2-Chloroacetamid, Chlorhexidin, Chlorhexidinacetat, Chlorhexidingluconat, Chlorhexidinhydrochloride, 1-Phenoxypropan-2-ol, N-alkyl(C12-C22)trimethylammoniumbromid und chlorid, 4,4-Dimethyl-1,3-oxazolidin, N-hydroxymethyl-N-(1,3-di(hydroxymethyl)-2,5-dioxoimidazolidin-4-yl)-N'-hydroxymethylharnstoff, 1,6-Bis(4-amidinophenoxy)-n-hexan und seine Salze, Glutaraldehyd, 5-Ethyl-1-aza-3,7-dioxabicyclo(3.3.0)octan, 3-(4-Chlorophenoxy)-1,2-propandiol, Hyamin, Alkyl-(C8-C18)-dimethylbenzyl-ammoniumchlorid, Alkyl-(C8-C18)- dimethylbenzylammoniumbromid, Alkyl-(C8-C18)-dimethylbenzylammoniumsaccharinat, Benzylhemiformal, o-Cymen-5-ol, Natriumhydroxymethylaminoacetat oder Natriumhydroxymethylaminoacetat, sowie Mischungen der vorstehend genannten Stoffe.

Weitere geeignete Konservierungsmittel umfassen Parabene. Parabene sind Ester der para-Hydroxybenzoesäure, wie beispielsweise Methylparaben, Ethylparaben, n-Propylparaben, n-Butylparaben, Isobutylparaben und Mischungen. Parabene können alleine oder in Kombination als Konservierungsmittel, insbesondere in kosmetischen und pharmazeutischen Zusammensetzungen verwendet werden. Eine bevorzugte Kombination von Parabenen bei den vorstehend genannten Zusammensetzungen umfasst 18 Gew.-% Methylparaben und 0.02 Gew.-% Propylparaben. Parabene weisen sowohl eine antimikrobielle als auf eine fungizide Wirkung auf.

Ferner sind erfindungsgemäß in der Kosmetik gebräuchliche Konservierungsmittel oder Konservierungshilfsstoffe geeignet, wie Dibromdicyanobutan (2-Brom-2-brommethylglutarodinitril), 3-Iod-2-propinylbutylcarbamat, 2-Brom-2-nitro-propan-1,3-diol, Imidazolidinylharnstoff, 5-Chlor-2-methyl-4-isothiazolin-3-on, 2-Chloracetamid, Benzalkoniumchlorid, Benzylalkohol und Formaldehydabspalter.

Außerdem sind Phenylhydroxyalkylether, insbesondere die unter der Bezeichnung Phenoxyethanol bekannte Verbindung, aufgrund ihrer bakteriziden und fungiziden Wirkungen auf eine Anzahl von Mikroorganismen als Konservierungsmittel geeignet.

Stabilisatoren werden im Rahmen der vorliegenden Erfindung dazu verwendet, die erfindungsgemäßen Zusammensetzungen gegenüber äußeren Umwelteinflüssen zu schützen. Derartige Umwelteinflüsse umfassen beispielsweise Luftsauerstoff und Lichteinstrahlung, insbesondere Sonnenlicht. Der oxidativen Wirkung durch Luftsauerstoff kann beispielsweise durch geeignete Antioxidationsmittel begegnet werden. Um die schädlichen Auswirkungen der Lichteinstrahlung zu begegnen können Fotostabilisatoren verwendet werden.

Die schützende Wirkung von einigen Benzoesäuren und ihren Salzen ist seit langem bekannt. Sie werden u.a. in Lebensmitteln vor allem zur mikrobiologischen Stabilisierung in sauren Anwendungen benutzt und sind als E 210 (Benzoesäure), E211 (Natriumbenzoat), E212 (Kaliumbenzoat) und E213 (Calciumbenzoat) bekannt. Ebenfalls werden verschiedene Parabene (Hydroxybenzoesäureester) unter den Nummern E 214 (Ethyl-p-hydroxybenzoat), E215 (Natriumethyl-p-hydroxybenzoat), E218 (Methyl-p-hydroxybenzoat) und E219 (Natriummethyl-p-hydroxybenzoat) in Lebensmitteln zur Verbesserung der mikrobiologischen Stabilität eingesetzt (BGBI. I 2000, 1537-1545). Auch in der Kosmetik findet diese Stoffklasse für die Zwecke der Verbesserung der (mikrobiologischen) Stabilität breite Anwendung.

Geeignete Stabilisatoren können außerdem beispielsweise der DE 299 24 656, DE 699 17 811, DE 696 24 799, EP 2 952 103 und der DE 695 18 581 entnommen werden.

Gemäß einer Ausführungsform erfolgt das Konzentrieren des Schafgarben Frischpflanzenpresssafts bei einer Temperatur von < 60°C.

Das Konzentrieren kann sowohl unter Normaldruck (1013 mbar) oder partiellem Unterdruck, beispielsweise 100 bis 1000 mbar, vorzugsweise 200 bis 500 mbar, erfolgen. Vorzugsweise erfolgt das Konzentrieren des Schafgarben Frischpflanzenpresssafts bei einer Temperatur von < 55°C, mehr bevorzugt < 50°C, < 45°C, < 40°C, < 35°C, < 30°C, < 25°C, noch mehr bevorzugt < 10°C, < 0°C, oder < 20°C. Die Kombination eines partiellen Unterdrucks mit einer niedrigen Temperatur ist aufgrund der schonenden Behandlung des Konzentrats besonders bevorzugt.

Gemäß einer Ausführungsform erfolgt das Konzentrieren des Schafgarben Frischpflanzenpresssafts durch ein Konzentrationsverfahren ausgewählt unter Gefriertrocknung, Sprühtrocknung und Umkehrosmose oder einer Kombination davon. Diese Verfahren sind im Stand der Technik gut bekannt.

Es hat sich gezeigt, dass v.a. die Umkehrosmose insbesondere aufgrund der schonenden Konzentrierung der wässrigen Lösungen zur Herstellung der erfindungsgemäßen Wasser-basierten Extrakt/Saft-Konzentrate von Vorteil ist. Die Umkehrosmose, die auch Hyperfiltration oder Reversosmose genannt wird, wird für gewöhnlich mit Membranen aus PAN (Polyacrylnitril), PES (Polyethersulfon) und/oder PVDF (Polyvinylidendifluorid) mit einer NaCl Ausschlussrate von $\geq$ 90 %, bevorzugt $\geq$ 95 %, $\geq$ 97 %, mehr bevorzugt 99 %, einem Betriebstrom von 8 l/m$^2$/h bis 34 l/m$^2$/h, vorzugsweise 8 l/m$^2$/h bis 25 l/m$^2$/h, 8 l/m$^2$/h bis 20 l/m$^2$/h Druck, mehr bevorzugt 8 l/m$^2$/h bis 15 l/m$^2$/h bei einem Druck von 15 bis 50 bar, vorzugsweise 15 bis 40 bar, mehr bevorzugt 15 bis 35 bar und einer Temperatur von $\leq$ 60 °C, vorzugsweise $\leq$ 50 °C, $\leq$ 40 °C, mehr bevorzugt $\leq$ 30 °C durchgeführt.

Es ist weiterhin von Vorteil, diese Konzentrate vor mikrobieller Kontamination zu schützen. Dies kann bspw. durch Absenken des pH Wertes und/oder Zusatz geeigneter Konservierungsmittel, wie vorstehend beschrieben, erreicht werden.

Die Gefriertrocknung, auch als Lyophilisierung, Lyophilisation oder Sublimationstrocknung bezeichnet, ist ein äußerst schonendes Verfahren, jedoch kostenintensiv und relativ aufwendig. Die Gefriertrocknung beruht auf dem physikalischen Prozess der Sublimation: Dabei sublimieren die Eiskristalle ohne zwischenzeitliches Auftreten einer flüssigen Phase direkt in den gasförmigen Zustand. Die Gefriertrocknung ergibt im vorliegenden Fall auch ohne Zusatz von Konservierungsmitteln einen gut lagerbaren Feststoff. Verfahren zur Gefriertrocknung können beispielsweise der GB 1 447 988 und DE 198 17 177 entnommen werden.

Bei der Sprühtrocknung, beispielsweise in der Wirbelschicht, wird das zu trocknende Gut mittels eines Zerstäubers in einen Heißgasstrom eingebracht, der es in sehr kurzer Zeit (wenige Sekunden bis Bruchteilen einer Sekunde) zu einem feinen Pulver trocknet. Die Sprühtrocknung kann wahlweise mit Zusatz der vorstehend genannten Trägerstoffe wie z.B. Maltodextrin, Cyclodextrin, usw., erfolgen. Von Vorteil ist hierbei, dass es sich um ein etabliertes Verfahren handelt, das kostengünstig durchgeführt werden kann. Lediglich eine sehr kurzzeitige Exposition, bspw. für wenige Sekunden, vorzugsweise weniger als 3 Sekunden oder weniger als 1 Sekunde, bei höheren Temperaturen, bspw. 200-400°C, vorzugsweise 250-270°C, ist von Nöten. Erhalten wird ein Feststoff, der auch ohne Zusatz von Konservierungsmitteln gut gelagert werden kann.

Vorzugsweise ist das Schafgarbe Frischpflanzenpresssaft Konzentrat durch das erfindungsgemäße Verfahren erhältlich.

Vorzugsweise ist das erfindungsgemäße Schafgarbe Frischpflanzenpresssaft Konzentrat herstellbar oder hergestellt durch ein Verfahren, das Sprühtrocknung umfasst.

Gemäß einer Ausführungsform wird eine kosmetische Zusammensetzung, pharmazeutische Zusammensetzung, Nahrungsmittelzusammensetzung oder Nahrungsergänzungsmittel bereitgestellt. Die pharmazeutische Zusammensetzung, Nahrungsmittelzusammensetzung oder das Nahrungsergänzungsmittel umfassen jeweils das erfindungsgemäße Schafgarbe Frischpflanzenpresssaft Konzentrat. Die kosmetische Zusammensetzung umfasst Schafgarbe Frischpflanzenpresssaft oder das erfindungsgemäße Schafgarbe Frischpflanzenpresssaft Konzentrat.

Die erfindungsgemäßen Konzentrate besitzen ein breites Anwendungsgebiet in der Humankosmetik und -pflege, insbesondere der Haut- und Haarpflege, sind aber auch pharmakologisch einsetzbar, sowie in Nahrungsmitteln und Nahrungsergänzungsmitteln verwendbar.

Bei den kosmetischen Zusammensetzungen kann es sich insbesondere um hautkosmetische, haarkosmetische, dermatologische und hygienische Zusammensetzungen handeln. Insbesondere werden die erfindungsgemäßen Konzentrate für die Haut- und/oder Haarkosmetik angewendet.

Die erfindungsgemäßen haar- oder hautpflegenden Zusammensetzungen bzw. Zubereitungen liegen vorzugsweise in Form einer Emulsion, einer Dispersion, einer Suspension, in Form einer wässrigen Tensidzubereitung, einer Milch, einer Lotion, einer Creme, eines Balsams, einer Salbe, eines Gels, eines Granulats, eines Puders, eines Stiftpräparates, wie z. B. eines Lippenstifts, eines Schaums, eines Aerosols oder eines Sprays vor. Solche Formulierungen sind gut geeignet für topische Zubereitungen. Als Emulsionen kommen Öl-in-Wasser-Emulsionen und Wasser-in-Öl-Emulsionen oder Mikroemulsionen in Frage.

Im Regelfall wird die haar- oder hautkosmetische Zubereitung zur Applikation auf der Haut oder dem Haar verwendet. Unter "topischen Zubereitungen" sind dabei solche Zubereitungen zu verstehen, die dazu geeignet sind, die Wirkstoffe in feiner Verteilung, wie z. B. in einer durch die Haut resorbierbaren Form auf die Haut aufzubringen. Hierfür eignen sich z. B. wässrige und wässrig-alkoholische Lösungen, Sprays, Schäume, Schaumaerosole, Salben, wässrige Gele, Emulsionen vom O/W- oder W/O-Typ, Mikroemulsionen oder kosmetische Stiftpräparate.

Die kosmetische Zusammensetzung kann einen (oder mehrere) Träger enthalten. Bevorzugt als Träger ist Wasser, ein Gas, eine Wasser-basierte Flüssigkeit, ein Öl, ein Gel, eine Emulsion oder Mikroemulsion, eine Dispersion oder eine Mischung davon. Die genannten Träger zeigen eine gute Hautverträglichkeit. Besonders vorteilhaft für topische Zubereitungen sind wässrige Gele, Emulsionen oder Mikroemulsionen. Der Trägerstoff kann ferner in der Form einer homogenen Phasenformulierung oder in Form einer Emulsion vorliegen. Bei der Emulsion kann es sich hierbei um eine Öl-in-Wasser, Wasser-in-Öl oder Mehrphasenemulsionen handeln. Diese Emulsionen können in einem weiten Konsistenzbereich, einschließlich dünnflüssiger Lotionen (die ebenso als Spray oder als Aerosol verabreicht werden können), cremige Lotionen, leichte und schwere Cremes und ähnliches, eingesetzt werden. Andere geeignete Trägerstoffe umfassen wasserfreie flüssige Lösungsmittel, wie beispielsweise Öle und Alkohole, wasserbasierende flüssige Lösungsmittel mit einer Phase (bspw. hydroalkoholische Lösungsmittelsysteme), wasserfrei Feststoffe und Semifeststoffe (wie beispielsweise Gele und Stifte) und wasserbasierende Gel- und Schaumsysteme. Beispiele für ihm Rahmen der vorliegenden Erfindung geeignete Trägerstoffe und Trägersystem können beispielsweise "Sun Products Formulary" Cosmetics & Toiletries, Vol. 105, S. 122-139 (Dezember 1990); "Sun Products Formulary", Cosmetics & Toiletries, Vol. 102, S. 117-136 (März 1987); der US 4,960,764, Figueroa et al.; und der US 4,254,105, Fukuda et al., entnommen werden.

Die erfindungsgemäße Lehre umfasst auch die Verwendung der hierin beschriebenen Konzentrate in einer pharmazeutischen Zusammensetzung. Diese pharmazeutische Zusammensetzung kann als Medikament eingesetzt werden, das zur Behandlung eines Individuums, vorzugsweise eines Säugers, insbesondere eines Menschen, dient. Die pharmazeutische Zusammensetzung umfasst für gewöhnlich einen pharmazeutisch verträglichen Exzipienten mit dem erfindungsgemäßen Konzentrat und gegebenenfalls einem oder mehreren anderen Wirkstoffen.

Diese Zusammensetzungen können beispielsweise auf oralem und transdermalem Weg verabreicht werden. Beispiele geeigneter pharmazeutischer Formulierungen sind feste Arzneiformen, wie Pulver, Puder, Granulate, Tabletten, Pastillen, Sachets, Cachets, Dragees, Kapseln wie Hart- und Weichgelatinekapseln, halbfeste Arzneiformen, wie Salben, Cremes, Hydrogele, Pasten oder Pflaster, sowie flüssige Arzneiformen, wie Lösungen, Emulsionen, insbesondere Öl-in-Wasser-Emulsionen, und Suspensionen, beispielsweise Lotionen.

Bei der Herstellung erfindungsgemäßer Zusammensetzungen werden erfindungsgemäß zu verwendende Bestandteile bzw. Wirkstoffe gewöhnlich mit einem Exzipienten vermischt oder verdünnt. Exzipienten können feste, halbfeste oder flüssige Materialien sein, die als Vehikel, Träger oder Medium für die in dem Konzentrat enthaltenen Wirkstoffe dienen. Der Wirkstoffgehalt (eines oder mehrerer gleichzeitig enthaltener erfindungsgemäßer Wirkstoffe) kann dabei in einem weiten Bereich variieren.

Zu geeigneten Exzipienten gehören beispielsweise Lactose, Dextrose, Sucrose, Sorbitol, Mannitol, Stärken, Akaziengummi, Calciumphosphat, Alginate, Traganth, Gelatine, Calciumsilikat, mikrokristalline Cellulose, Polyvinylpyrrolidon, Cellulose, Wasser, Sirup und Methylcellulose. Ferner können die Formulierungen pharmazeutisch akzeptable Träger oder übliche Hilfsstoffe, wie Gleitmittel, beispielsweise Talg, Magnesiumstearat und Mineralöl; Netzmittel; emulgierende und suspendierende Mittel; konservierende Mittel, wie Methyl und Propylhydroxybenzoate; Antioxidantien; Antireizstoffe; Chelatbildner; Dragierhilfsmittel; Emulsionsstabilisatoren Filmbildner; Gelbildner; Geruchsmaskierungsmittel; Geschmackskorrigentien; Harze; Hydrokolloide; Lösemittel; Lösungsvermittler; Neutralisierungsmittel; Permeationsbeschleuniger; Pigmente; quaternäre Ammoniumverbindungen; Rückfettungs- und Überfettungsmittel; Salben-, Creme- oder Öl-Grundstoffe; Silikon-Derivate; Spreithilfsmittel; Stabilisatoren; Sterilanzien; Suppositoriengrundlagen; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle umfassen. Eine diesbezügliche Ausgestaltung beruht auf fachmännischem Wissen, wie beispielsweise in Fiedler, H. P., Lexikon der Hilfsstoffe für Pharmazie, Kosmetik und angrenzende Gebiete, 4. Auflage, Aulendorf: ECV-Editio-Kantor-Verlag, 1996, oder Hager's Handbuch der Pharmazeutischen Praxis, Springer Verlag, Heidelberg dargestellt ist.

Im Rahmen der vorliegenden Erfindung ist es grundsätzlich möglich und regelmäßig vorteilhaft, den erfindungsgemäßen bzw. erfindungsgemäß einzusetzenden Frischpflanzenpresssaft oder das Konzentrat mit weiteren (anderen) (Wirk-)Stoffen zu kombinieren, z.B. ausgewählt aus der Gruppe bestehend aus Abrasiva, Antiakne-Mittel und Mittel zu Sebumreduktion, Mittel gegen Hautalterung, antibakterielle Mittel, Anticellulitis-Mittel, Antischuppen-Mittel, entzündungshemmende Mittel, irritationsverhindernde Mittel, Antiirritantien (antiinflammatorische, irritationshemmende und irritationsverhindernde Mittel), antimikrobielle Mittel, Antioxidantien, Adstringentien, antiseptische Mittel, Antistatika, Binder, Puffer, Trägermaterialien, Chelatbildnder, Zellstimulantien, reinigende Mittel, pflegende Mittel, Enthaarungsmittel, oberflächenaktive Substanzen, deodorierende Mittel und Antiperspirantien, Weichmacher, Emulgatoren, Enzyme, ätherische Öle, Insektenrepellentien, Fasern, Filmbildner, Fixateure, Schaumbildner, Schaumstabilisatoren, Substanzen zum Verhindern des Schäumens, Schaumbooster, Fungizide, gelierende Mittel und gelbildende Mittel, Haarpflegemittel, Haarverformungsmittel, Haarglättungsmittel, Feuchtigkeitsregulatoren (feuchtigkeitsspendende, anfeuchtende und/oder feuchthaltende Substanzen), Osmolyte, kompatible Solute, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine und Proteinhydrolysate, rückfettende Mittel, abschleifende Mittel, hautberuhigende Mittel, hautreinigende Mittel, hautpflegende Mittel, hautheilende Mittel (skin repair agents), vorzugsweise enthaltend Cholesterin und/oder Fettsäuren und/oder Ceramide und/oder Pseudoceramide, Hautaufhellungsmittel, hautschützende Mittel, hauterweichende Mittel, hautkühlende Mittel, vorzugsweise die in WO 2005/123101 genannten, hautwärmende Mittel, UV-absorbierende Mittel und UV-Filter, vorzugsweise die in WO 2005/123101 genannten, Benzyliden-beta-dicarbonylverbindungen, alpha-Benzoylzimtsäurenitrile, AhR-Rezeptor-Antagonisten, Waschmittel, Weichspüler, suspendierende Mittel, Hautbräunungsmittel, Verdickungsmittel, Vitamine, genannten, Öle, Wachse und Fette, Phospholipide, Fettsäuren (gesättigte Fettsäuren, ein- oder mehrfach ungesättigte Fettsäuren, $\alpha$-Hydroxysäuren, Polyhydroxyfettsäuren), Verflüssiger, Farbstoffe und farbschützende Mittel sowie Pigmente, Antikorrosiva, Aromen und Geschmackstoffe sowie Riechstoffe, vorzugsweise die in S. Arctander, Perfume and Flavor Chemicals, Eigenverlag, Montclair, N.J., 1969 und Surburg, Panten, Common Fragrance and Flavor Materials, 5th Edition, Wiley-VCH, Weinheim 2006 aufgeführten, Tenside, tierische Extrakte, Hefeextrakte, Extrakte von Algen oder Mikroalgen, Elektrolyte, Verflüssiger, organische Lösungsmittel, haarwachstumsmodulierende Mittel (haarwachstumsfördend oder haarwachstumshemmend) und Silikone und Silikonderivate,

bevorzugt einem oder mehreren hautkühlenden Mitteln und/oder einem oder mehreren UV-absorbierenden Mitteln oder UV-Filtern.

Die erfindungsgemäßen Zusammensetzungen können demnach neben üblichen Zusatzoder Hilfsstoffen zusätzlich auch (weitere) kosmetisch und/oder dermatologisch und/oder pharmakologisch aktive Wirkstoffe enthalten.

Geeignete kosmetisch und/oder dermatologisch aktive Wirkstoffe sind z. B. färbende Wirkstoffe, Haut- und Haarpigmentierungsmittel, Tönungsmittel, Bräunungsmittel, Bleichmittel, keratinhärtende Stoffe, antimikrobielle Wirkstoffe, Lichtfilternde Wirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder -feuchthaltende Stoffe, rückfettende Wirkstoffe, antierythimatös oder antiallergisch aktive Wirkstoffe, verzweigte Fettsäuren, wie 18-Methyleicosansäure, und Mischungen davon.

Künstlich hautbräunende Wirkstoffe, die geeignet sind, die Haut ohne natürliche oder künstliche Bestrahlung mit UV-Strahlen zu bräunen; dies sind z. B. Dihydroxyaceton, Alloxan und Walnussschalenextrakt. Geeignete keratinhärtende Stoffe sind in der Regel Wirkstoffe, wie sie auch in Antitranspirantien eingesetzt werden, wie z. B. Kaliumaluminiumsulfat, Aluminiumhydroxychlorid, Aluminiumlactat etc.

Antimikrobielle Wirkstoffe, die eingesetzt werden, um Mikroorganismen zu zerstören bzw. ihr Wachstum zu hemmen. Sie dienen somit sowohl als Konservierungsmittel als auch als desodorierend wirkender Stoff, welcher die Entstehung oder die Intensität von Körpergeruch vermindert. Dazu zählen z. B. übliche, dem Fachmann bekannte Konservierungsmittel, wie p-Hydroxybenzoesäureester, Imidazolidinyl-Harnstoff, Formaldehyd, Sorbinsäure, Benzoesäure, Salicylsäure etc. Derartige desodorierend wirkende Stoffe sind z. B. Zinkricinoleat, Triclosan, Undecylensäurealkylolamide, Citronensäuretriethylester, Chlorhexidin etc.

Geeignete Hilfs- und Zusatzstoffe für die Herstellung von haarkosmetischen oder hautkosmetischen Zubereitungen sind dem Fachmann geläufig und können aus Handbüchern der Kosmetik, beispielsweise Schrader, Grundlagen und Rezepturen der Kosmetika, Hüthig Verlag, Heidelberg, 1989, ISBN 3-7785-1491-1, entnommen werden. Die Hilfsund Zusatzstoffe sind bevorzugt kosmetisch und/oder pharmazeutisch akzeptable Hilfsstoffe. Pharmazeutisch akzeptabel sind die im Bereich der Pharmazie, der Lebensmitteltechnologie und angrenzenden Gebieten bekanntermaßen anwendbaren Hilfsstoffe, insbesondere die in einschlägigen Arzneibüchern (z. B. DAB, Ph. Eur., BP, NF) gelisteten sowie andere Hilfsstoffe, deren Eigenschaften einem physiologischen Anwendung nicht entgegenstehen.

Geeignete Hilfsstoffe können sein: Gleitmittel, Netzmittel, emulgierende und suspendierende Mittel, konservierende Mittel, Antioxidanzien, Antireizstoffe, Chelatbildner, Emulsionsstabilisatoren, Filmbildner, Gelbildner, Geruchsmaskierungsmittel, Hydrokolloide, Lösemittel, Lösungsvermittler, Neutralisierungsmittel, Permeationsbeschleuniger, Pigmente, quaternäre Ammoniumverbindungen, Rückfettungs- und Überfettungsmittel, Salben-, Creme- oder Öl-Grundstoffe, Silikonderivate, Stabilisatoren, Sterilanzien, Treibmittel, Trocknungsmittel, Trübungsmittel, Verdickungsmittel, Wachse, Weichmacher, und Weißöl.

Weitere geeignete Zusatzstoffe sind ausgewählt unter Parfümölen, Haarpolymeren, Haar- und Hautkonditionern, Pfropfpolymeren, wasserlöslichen oder dispergierbaren silikonhaltigen Polymeren, Lichtschutzmitteln, Bleichmitteln, Pflegemitteln, Färbemitteln, Tönungsmitteln, Bräunungsmitteln, Farbstoffen, Konsistenzgebern, Feuchthaltemitteln, Rückfettern, Collagen, Eiweißhydrolysaten, Lipiden, Antioxidanzien, Entschäumern, Antistatika, Emollienzien, Weichmachern, Peroxidzersetzern.

Als Beispiele geeigneter Hilfs- und Zusatzstoffe sind die vorstehend aufgeführten Antioxidanzien, Peroxidzersetzer, Verdickungsmittel und die vorstehend aufgeführten Konservierungsmittel aufzuführen.

Peroxidzersetzer sind Verbindungen die Peroxide, besonders bevorzugt Lipidperoxide, chemisch zu zersetzen.

Beispiele umfassen Pyridin-2-thiol-3-carbonsäure, 2-Methoxypyrimidinolcarbonsäuren, 2-Methoxy-pyridincarbonsäuren, 2-Dimethylamino-pyrimidinolcarbonsäuren, 2-Dimethylamino-pyridincarbonsäuren.

Beispielhafte Verdickungsmittel sind vernetzte Polyacrylsäuren und deren Derivate, Polysaccharide und deren Derivate, wie Xanthangummi, Agar-Agar, Alginate oder Tylosen, Cellulosederivate, z. B. Carboxymethylcellulose oder Hydroxycarboxymethylcellulose, Fettalkohole, Monoglyceride und Fettsäuren, Polyvinylalkohol und Polyvinylpyrrolidon. Insbesondere werden nichtionische Verdicker eingesetzt.

Neben den Konservierungsmitteln können auch andere keimhemmende Mittel zur Anwendung gelangen und in die erfindungsgemäßen Zusammensetzungen eingearbeitet werden. Vorteilhafte Substanzen sind zum Beispiel 2,4,4'-Trichlor-2'-hydroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatriän-1-ol) sowie die in den Patentoffenlegungsschriften DE 37 40 186, DE 39 38 140, DE 42 04 321, DE 42 29 707, DE 43 09 372, DE-44 11 664, DE 195 41 967, DE 195 43 695, DE 195 43 696, DE 195 47 160, DE 196 02 108, DE 196 02 110, DE 196 02 111, DE 196 31 003, DE 196 31 004 und D -196 34 019 und den Patentschriften DE 42 29 737, DE 42 37 081, DE 43 24 219, DE 44 29 467, DE 44 23 410 und DE 195 16 705 beschriebenen Wirkstoffe bzw. Wirkstoffkombinationen. Auch Natriumhydrogencarbonat ist vorteilhaft zu verwenden. Ebenso können auch antimikrobielle Polypeptide eingesetzt werden.

Lichtfilternde Wirkstoffe können ebenfalls enthalten sein. Diese Wirkstoffe absorbieren die UV-Strahlen im UV-B- und/oder UV-A-Bereich. Geeignete UV-Filter sind z. B. 2,4,6-Triaryl-1,3,5-triazine, bei denen die Arylgruppen jeweils wenigstens einen Substituenten tragen können, der vorzugsweise ausgewählt ist unter Hydroxy, Alkoxy, speziell Methoxy, Alkoxycarbonyl, speziell Methoxycarbonyl und Ethoxycarbonyl, und Mischungen davon. Geeignet sind weiterhin p-Aminobenzoesäureester, Zimtsäureester, Benzophenone, Kampferderivate sowie UV-Strahlen abhaltende Pigmente, wie Titandioxid, Talkum und Zinkoxid.

Als UV-Filtersubstanzen kommen beliebige UV-A- und UV-B-Filtersubstanzen in Betracht. Beispielsweise sind zu nennen:

Die erfindungsgemäßen kosmetischen und dermatologischen Zubereitungen können vorteilhafterweise außerdem UV-Strahlen abhaltende anorganische Pigmente auf Basis von Metalloxiden und/oder anderen in Wasser schwer löslichen oder unlöslichen Metallverbindungen, ausgewählt aus der Gruppe der Oxide des Zinks (ZnO), Titan (TiOz), Eisens (z. B. $Fe_2O_3$), Zirkoniums (ZrOz), Siliciums (SiOz), Mangans (z. B. MnO), Aluminiums ($Al_2O_3$), Cers (z. B. $Ce_2O_3$), Mischoxiden der entsprechenden Metalle sowie Abmischungen aus solchen Oxiden enthalten.

Geeignete hyperemisierend wirkende Stoffe können ebenfalls in den kosmetischen oder pharmazeutischen Zusammensetzungen enthalten sein. Diese regen die Durchblutung der Haut an. Beispiele hierfür sind ätherische Öle, wie Latschenkieferextrakt, Lavendelextrakt, Rosmarinextrakt, Wacholderbeerextrakt, Rosskastanienextrakt, Birkenblätterextrakt, Heublumenextrakt, Ethylacetat, Kampfer, Menthol, Pfefferminzöl, Rosmarinextrakt, Eukalyptusöl etc.

Ebenso können keratolytisch und keratoplastisch wirkende Stoffe enthalten sein. Beispiele derartiger Stoffe umfassen Salicylsäure, Kalziumthioglykolat, Thioglykolsäure und ihre Salze, Schwefel etc. Geeignete Antischuppen-Wirkstoffe sind z. B. Schwefel, Schwefelpolyethylenglykolsorbitanmonooleat, Schwefelricinolpolyethoxylat, Zinkpyrithion, Aluminiumpyrithion etc.

Geeignete Antiphlogistika, die Hautreizungen entgegenwirken, sind z. B. Allantoin, Bisabolol, Dragosantol, Kamillenextrakt, Panthenol etc.

Kosmetisch oder pharmazeutisch akzeptable Polymere, wie kationische, amphotere und neutrale Polymere. Geeignete Polymere sind z. B. kationische Polymere mit der Bezeichnung Polyquaternium nach INCI z. B. Copolymere aus Vinylpyrrolidon/N-Vinylimidazoliumsalzen (Luviquat FC, Luviquat HM, Luviquat MS, Luviquat Care), Copolymere aus N-Vinylpyrrolidon/Dimethylaminoethylmethacrylat, quaternisiert mit Diethylsulfat (Luviquat PQ 11), Copolymere aus N-Vinylcaprolactam/N-Vinylpyrrolidon/N-Vinylimidazoliumsalzen (Luviquat E Hold), kationische Cellulosederivate (Polyquaternium-4 und -10), Acrylamidocopolymere (Polyquaternium-7) und Chitosan.

Geeignete kationische (quaternisierte) Polymere sind auch Merquat (Polymer auf Basis von Dimethyldiallylammoniumchlorid),Gafquat (quaternäre Polymere, die durch Reaktion von Polyvinylpyrrolidon mit quaternären Ammoniumverbindungen entstehen), Polymer JR (Hydroxyethylcellulose mit kationischen Gruppen) und kationische Polymere auf pflanzlicher Basis, z. B. Guarpolymere, wie die Jaguar-Marken der Firma Rhodia.

Weitere geeignete Polymere sind auch neutrale Polymere, wie Polyvinylpyrrolidone, Copolymere aus N-Vinylpyrrolidon und Vinylacetat und/oder Vinylpropionat, Polysiloxane, Polyvinylcaprolactam und andere Copolymere mit N-Vinylpyrrolidon, Polyethylenimine und deren Salze, Polyvinylamine und deren Salze, Cellulosederivate, Polyasparaginsäuresalze und deren Derivate. Dazu zählt beispielsweise Luviflex® Swing (teilverseiftes Copolymerisat von Polyvinylacetat und Polyethylenglykol, Firma BASF).

Geeignete Polymere sind auch nichtionische, wasserlösliche bzw. wasserdispergierbare Polymere oder Oligomere, wie Polyvinylcaprolactam, z. B. Luviskol® Plus (BASF), oder Polyvinylpyrrolidon und deren Copolymere, insbesondere mit Vinylestern, wie Vinylacetat, z. B. Luviskol® VA 37 (BASF), Polyamide, z. B. auf Basis von Itaconsäure und aliphatischen Diaminen, wie sie z. B. in der DE-A-43 33 238 beschrieben sind.

Geeignete Polymere sind auch amphotere oder zwitterionische Polymere, wie die unter den Bezeichnungen Amphomer (National Starch) erhältlichen Octylacrylamid/Methylmethacrylat/tert.-Butylaminoethylmethacrylat -Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 3708 451 offenbart sind. Acrylamidopropyltrimethylammoniumchlorid/Acrylsäure- bzw. Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette (AMERCHOL) im Handel erhältlich sind, und Copolymere aus Hydroxyethylmethacrylat, Methylmethacrylat, N,N-Dimethylaminoethylmethacrylat und Acrylsäure (Jordapon (D)). Geeignete Polymere sind auch nichtionische, siloxanhaltige, wasserlösliche oder dispergierbare Polymere, z. B. Polyethersiloxane, wie Tegopren® (Firma Goldschmidt) oder Besi (Firma Wacker).

Ein weiterer Gegenstand der Erfindung betrifft Nahrungs- oder Nahrungsergänzungsmittel, die die erfindungsgemäßen Konzentrate enthalten.

Soweit die konfektionierten Produkte Nahrungsmittel darstellen, denen man die Konzentrate direkt zusetzt, handelt es sich um Lebensmittel jeglicher Art. Beispielsweise sind Backwaren, alkoholische oder nicht-alkoholische Getränke, fruchthaltige Limonaden, (carbonisierte) isotonische Getränke, (carbonisierte) Erfrischungsgetränke, Nektare, Schorlen, Obst- und Gemüsesäfte, m Frucht- oder Gemüsesaftzubereitungen und Milchprodukte zu nennen. Vorzugsweise handelt es sich um Lebensmittel, die bei der Zubereitung nicht erhitzt werden müssen.

Handelt es sich bei den Produkten um Nahrungsmittelergänzungsstoffe, so werden diese in der Regel ohne weitere Zusatzstoffe eingesetzt, wobei von reinen Konfektionierungsmittel abzusehen ist. Eine bevorzugte Anwendungsform stellen hier Makro- oder Mikrokapseln dar. Makrokapseln bestehen vorzugsweise aus Gelatine oder es handelt sich um sprühgetrocknete Produkte, die als Basis Polysaccharide oder Dextrine enthalten.

Gemäß einer Ausführungsform umfassen die kosmetische Zusammensetzung oder die pharmazeutische Zusammensetzung weiter 0,01-10 Gew.-% eines hautberuhigenden Mittels und/oder eines Antioxidans.

Beispiele hautberuhigender Mittel oder Emollientia, die für eine topische Zusammensetzung geeignet sind, umfassen Bisabolol, Allantoin, Menthol, Saccharose, Centellaextrakt, Glycyrrhizaextrakt, Farnesol, Ruscusextrakt, Malvenextrakt, Jasminextrakt, Rosmarinsäure und Rosmarinsäure enthaltende Pflanzenextrakte, Haferextrakt, Ingwerextrakt, [6]-Paradol, Hamamelisextrakt, Calendulaextrakt, Arnikaextrakt, Sonnenhutextrakt, hydriertes Polyisobuten, C-12-C15-Alkoholbenzoate, Isopropylmyristat, Mineralöle, Lanolin und Lanolinderivate, Triglyceride, wie beispielsweise Kokosnussöl, Cetostearylalkohol, Cetylalkohol, Isopropylpalmitat, Capryl/Caprin Triglycerid, PPG-2 Myristyletherpropionat, Dimethicon, Methicon, Vaseline und andere dem Fachmann geläufige hautberuhigende Mittel.

Antioxidantien sind vorzugsweise ausgewählt unter Aminosäuren (z. B. Glycin, Histidin, Tyrosin, Tryptophan) und deren Derivaten, Imidazolen (z. B. Urocaninsäure) und deren Derivaten, Peptiden wie D,L-Camosin, D-Carnosin, L-Carnosin und deren Derivaten (z. B. Anserin), Carotinoiden, Carotinen (z. B. $\beta$-Carotin, Lycopin) und deren Derivaten, Chlorogensäure und deren Derivaten, Liponsäure und deren Derivaten (z. B. Dihydroliponsäure), Aurothioglucose, Propylthiouracil und anderen Thiolen (z. B. Thiorodoxin, Glutathion, Cystein, Cystin, Cystamin und deren Glycosyl-, N-Acetyl-, Methyl-, Ethyl-, Propyl-, Amyl-, Butyl-, und Lauryl-, Palmitoyl-, Oleyl-, y-Linoleyl-, Cholesteryl- und Glycerylester) sowie deren Salzen, Dilaurylthiodipropionat, Distearylthiodipropionat, Thiodipropionsäure und deren Derivaten (Ester, Ether, Peptide, Lipide, Nukleotide, Nukleoside und Salze) sowie Sulfoximinverbindungen (z. B. Buthioninsulfoximine, Homocysteinsulfoximine, Buthioninsulfone, Penta-, Hexa-, Heptathioninsulfoximin), insbesondere in sehrgeringen, verträglichen Dosierungen (z.B. pmol bis umol/kg Bereich), ferner (Metall)-Chelatoren (z. B. d-Hydroxyfettsäuren, Palmitinsäure, Phytinsäure,Lactoferrin), o-Hydroxysäuren (z. B. Citronensäure, Milchsäure, Apfelsäure), Huminsäure, Gallensäure, Gallenextrakte, Bilirubin, Biliverdin, EDTA und deren Derivaten, ungesättigten Fettsäuren und deren Derivaten (z. B. v-Linolensäure, Linolsäure, Ölsäure), Folsäure und deren Derivaten, Ubichinon und Ubichinolv und deren Derivaten, Vitamin C und dessen Derivaten (z. B. Natriumascorbat, Ascorbylpalmitat, Mg-Ascorbylphosphat, Ascorbylacetat), Tocopherol und Derivate (z. B. Vitamin-E-Acetat, Tocotrienol), Vitamin A und Derivate (Vitamin-A-Palmitat) sowie Koniferylbenzoat des Benzoeharzes, Rutinsäure und deren Derivaten, o-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, [6]-Paradol, 4-Hydroxyacetophenon, Harnsäure und deren Derivaten, Mannose und deren Derivaten, Zink und dessen Derivaten (z. B. ZnO, ZnSO4), Selen und dessen Derivaten (z. B. Selenmethionin), Stilbenen und deren Derivaten (z. B. Stilbenoxid, Trans-Stilbenoxid).

Gemäß einer Ausführungsform wird die kosmetische, nicht-therapeutische Verwendung von Schafgarbe zur Stimulierung der Expression von Hitzeschockproteinen (HSP) in Hautzellen bereitgestellt.

Die menschliche Haut ist ein Grenzorgan und stellt den Schutz vor u.a. Umwelteinflüssen bereit. Die menschliche Haut ist ständig einer großen Vielzahl potentiell gefährlicher Mikroorganismen ausgesetzt. Die Haut ist trotz dieser Bedrohung durch Mikroorganismen gegen Infektionen äußerst widerstandsfähig. Verschiedene im letzten Jahrzehnt durchgeführte Studien zeigen ein chemisches, in der Haut vorliegendes Verteidigungssystem auf, das auf der Herstellung antimikrobieller Peptide und Proteine (AMPs) beruht. Die AMPs stellen eine vielfältige Gruppe kleiner Moleküle (12-100 Aminosäurereste) dar, die das Haupteffektorsystem der angeborenen Immunität ausmachen. Sie stellen die erste Verteidigungslinie gegenüber Pathogenen bereit. AMPs, die in Reaktion auf eine Gefahr erzeugt werden, können innerhalb kurzer Zeit ein breites Spektrum der Mikroben, wie beispielsweise Bakterien, Pilze, Viren oder Protozoen, durch ihr breites Spektrum an wirksamer antimikrobieller Aktivität bereitstellen. AMPs wurden in einer Vielzahl exponierter Gewebe oder Oberflächen, wie beispielsweise Haut, Augen, Ohren, Mund, Atemwege, Lunge, Eingeweiden, und dem Harntrakt, identifiziert. In der menschlichen Haut können sie sowohl durch ansässige Hautzellen als auch infiltrierende Immunzellen entweder konstitutiv oder in Reaktion auf eine Gefahr, wie beispielsweise eine Infektion, Trauma, Wundheilung oder chronische Entzündung produziert werden. AMPs werden in der menschlichen Haut hauptsächlich durch Keratinozyten, Neutrophile, Sebozyten oder Schweißdrüsen produziert. Bei mehreren Erkrankungen der menschlichen Haut besteht eine reziproke Korrelation zwischen der Schwere der Erkrankung und dem Niveau einer AMP Erzeugung. Hautverletzungen von Patienten mit atopischer Dermatitis zeigen eine verminderte Expression der Defensine und des Cathelicidines LL-37. Erhöhte Niveaus an AMPs hängen darüberhinaus mit Verbrennungen und chronischen Wunden zusammen. Dem gegenüber kann eine Überexpression von AMPs zu einem erhöhten Schutz gegenüber Hautinfektionen führen, wie es bei Patienten mit Psoriasis und Rosacea und entzündlichen Hautinfektionen beobachtet wird, die selten zu einer Superinfektion führen. Die alpha und beta Familien umfassenden Defensine stellen eine der größten und meist studierten Familien der AMPs in Säugern dar. Sie werden von einer Vielzahl von Haut und Knochenmark abgeleiteten Zellen produziert, weisen ein breites Spektrum einer antimikrobiellen Aktivität gegenüber gram-positiven und gram-negativen Bakterien, Viren, Pilzen und einigen Protozoen auf und stellen wichtige Bestandteile des angeborenen Immunsystems dar. Beta Defensine sind kationische Peptide mit antimikrobieller Aktivität, die Epitheloberflächen einschließlich der Haut, Gastrointestinal-, Harntrakt und Atemwege verteidigen. Menschliches β-Defensin 1 Peptid (hBD-1) wird von dem DEFB1 Locus kodiert und wirkt gegenüber gram-positiven und negativen Bakterien. Nach der Reduktion der Disulfidbrücken wird hBD-1 ein wirksames AMP gegenüber dem opportunistischen pathogenen Pilz Candida albicans. Es zeigt eine synergistische Wirkung mit LL-37 oder Lysozym gegenüber S. aureus und E. coli.

S100 Proteine sind kationische Proteine mit niedrigem Molekulargewicht und durch zwei Calciumbindende EF-Hand Motive gekennzeichnet. Sie sind in einer Vielzahlzahl zellulärer Vorgänge, wie beispielsweise die calciumabhängige Zellsignalgebung, Zellwachstum, und Verteidigung gegenüber Mikroben beteiligt. S100 Calcium-Bindeprotein A8 (S100A8) ist ein Protein, dass durch das S100A8 Gen im Menschen codiert wird. S100A8 (Calgranulin A) und S100A9 (Calgranulin B) bilden einen antimikrobiellen, heterodimeren Komplex, der ebenso als Calprotectin, in epidermalen Keratinozyten bekannt ist. Es wurde gezeigt, dass der S100A8/S100A9 Komplex ebenso eine antimikrobielle Aktivität gegenüber Bakterien, wie beispielsweise Escherichia coli, Klebsiella spp., Staphylococcus aureus, S. epidermis, Capnocytophaga sputigena und Borrelia burgdorferi ebenso wie eine pilzhemmende Aktivität gegenüber dem Pilz Candida albicans, aufweist.

Hitzeschockproteine (HSPs) sind molekulare Chaperone, die den Zelltod verhindern. Organismen und Zellen reagieren auf verschiedene Stressbedingungen, wie beispielsweise Umweltbedingter, metabolischer und pathophysiologischer Stress, durch selektive Hochregulierung der HSPs. Sie werden mittels ihrer erhöhten Expression nach einem Hitzeschock (im Normalfall eine Stunde oder mehr bei Temperaturen von 3-5°C über der gewöhnlichen) identifiziert. Die Annahme, dass HSPs Zellen gegenüber Hitzeschädigung schützen, wird durch die folgenden Tatsachen gestützt: 1) die HSP Expression erfolgt genau parallel zu der Entwicklung und dem Abfall der Thermotoleranz (Widerstand gegenüber Abtötung durch Hitze); 2) Mutation oder Inaktivierung der HSPs beeinträchtigt die Befähigung einer Zelle, bei hohen Temperaturen zu überleben; 3) Überexpression von HSPs kann häufig die Befähigung einer Zelle verbessern, hohen Temperaturen zu widerstehen. Die Induktion des Hitzeschockproteins HSP70 in humanen Keratinozyten und NIH/3T3 Fibroblasten durch Zimtsäure-Derivate, u.a. Chlorogensäure, wird beispielsweise in der US 9265743 beschrieben.

Diese Proteine sind auf Grundlage ihres Molekulargewichts in sechs Hauptfamilien, d.h. Hsp100, Hsp90, Hsp70, Hsp60, Hsp40 und kleine Hitzeschockproteine (sHsps) klassifiziert. sHsps weisen Untereinheiten-Molekulargewichte von 12-43 kDa auf. Die Befähigung Aggregation von Proteinen und Polypeptiden, insbesondere unter Stressbedingungen, die zu einem Entfalten der zellulären Proteine führen, zu verhindern, ist die wichtigste Funktion vieler sHSPs. Menschliche sHSPs weisen äußerst verschiedene Merkmale im Hinblick auf ihre hitzeinduzierte Expression, Gewebe und Intrazelluläre Lokalsierung, Aufbau, Substratpräferenz und Funktion auf. Aufgrund dieses Unterschieds weisen menschliche sHSPs unterschiedliche Befähigungen zum Schutz gegenüber akutem und unterschiedlichen Arten von chronischem (Erkrankungsbezogenen) Stress auf.

HSP27 gehört zu den kleinen Hitzeschockproteine(sHSPs). Es ist ein ATP-unabhängiges molekulares Chaperon, das gegenüber Stress-induzierter Aggregation von Proteinen schützt. Hsp27 befindet sich in intakter Haut hauptsächlich in der Körner- und Stachelzellschicht der Epidermis.

Gemäß einer Ausführungsform wird die kosmetische, nicht-therapeutische Verwendung von Schafgarbe zur Erhöhung der Thermotoleranz der Haut bereitgestellt.

Die Thermotoleranz der Haut betrifft ein induzierbares Phänomen, bei dem Zellen nach einem Hitzestress resistent gegenüber einer sonst schädigenden oder letalen Hitzebelastung werden. Aus Maytin E. et al. (J. Invest. Dermatol 1990;95: 635-42;J. Biol Chem. 1992; 267: 231 89-96; J. Invest Dermatol 1995; 104 [4]: 448-55, Farage MA.

Textbook of Aging Skin. Springer-Verlag Berlin Heidelberg 2010) kann entnommen werden, dass eine Hitzebelastung von menschlichen Keratinozyten nicht nur zu einer Thermotoleranz und zu einem Anstieg der Hitzeschockproteinsynthese, sondern auch zu einer Unempfindlichkeit gegenüber anderen belastenden Reizen wie Schwermetalle, Hypoxie und überraschenderweise auch zu einer Resistenz gegenüber UVB-induzierten Schäden führt.

Gemäß einer Ausführungsform wird bei der erfindungsgemäßen Verwendung die Schafgarbe als Schafgarbe Frischpflanzenpresssaft oder als erfindungsgemäße kosmetische Zusammensetzung eingesetzt.

Gemäß einer Ausführungsform beträgt bei der erfindungsgemäßen Verwendung die Dosierung von Schafgarbe Frischpflanzenpresssaft Konzentrat in der kosmetischen Zusammensetzung 0.0001 - 10 Gew.-% bezogen auf 100 Gew.-% der kosmetischen Zusammensetzung.

Vorzugsweise beträgt die Dosierung von Schafgarbe Frischpflanzenpresssaft Konzentrat in kosmetischen Zusammensetzungen, insbesondere in kosmetischen Zusammensetzung zur Reinigung und Pflege von Haut und Haar, 0.001-5 Gew.-% und mehr bevorzugt 0.005-3 Gew.-%.

Gemäß einer Ausführungsform wird die erfindungsgemäße pharmazeutische Zusammensetzung als Medikament verwendet.

Die erfindungsgemäße pharmazeutische Zusammensetzung kann ebenfalls bei der Krebsprävention und bei der Behandlung von Hauterkrankungen Anwendung finden.

Es ist bekannt, dass bei Erkrankungen wie atopischer Dermatitis, aktinischen Keratose, einer Vorstufe von Hautkrebs, Psoriasis, Rosacea und anderen Hauterkrankungen, die durch Barriereschäden und chronische Entzündungen charakterisiert sind, die Hitzeschockproteinsynthese in den entsprechenden Hautregionen vermindert ist und die Reparaturmechanismen der Haut gestört sind. In der kontrollierten Apoptose der Keratinozyten treten Störungen auf und die Immunabwehr wird geschwächt. Parallel zu diesen Vorgängen werden vermehrt inflammatorische Enzyme wie die Matrix-Metalloproteinasen gebildet. Hautalterung und -entzündung sind dadurch gekennzeichnet, dass die Reparaturmechanismen der Haut gestört und geschwächt sind und Entzündungsmediatoren vermehrt freigesetzt werden.

Insbesondere kann die erfindungsgemäße pharmazeutische Zusammensetzung zum Vermeiden und/oder Lindern von Hautschädigungen im Rahmen einer Krebstherapie verwendet werden. Diese Verwendung der erfindungsgemäßen pharmazeutische Zusammensetzung beruht auf der vorgefunden Wirkungsweise der Schafgarbe, insbesondere der Inhaltsstoffe der Schafgarbe, die eine Erhöhung der Thermotoleranz in der Haut bewirken. Es kann angenommen werden, dass diese Wirkung in der Expression von Hitzeschockproteinen in Hautzellen beruht. Dieser Wirkung kann dazu genutzt werden, um im Rahmen der Krebstheraphie, insbesondere der Radiotherapie von Hautkrebs, gesunde Hautzellen vor den medikamentösen und/oder strahlungsbedingten Auswirkungen zu schützen. Die antimikrobiellen Eigenschaften der Schafgarbe unterstützen ferner diese Schutzwirkung für die menschliche Haut.

Gemäß einer Ausführungsform erfolgt bei der erfindungsgemäßen Verwendung von Schafgarbe oder der erfindungsgemäßen Verwendung der pharmazeutischen Zusammensetzung zur Verwendung als Medikament, eine Anwendung topisch auf die Haut und/oder die Haare.

Der Ausdruck "aufweisen" bzw. "aufweisend" bezeichnet im Rahmen der vorliegenden Erfindung eine offene Aufzählung und schließt neben den ausdrücklich genannten Bestandteilen bzw. Schritten andere Bestandteile bzw. Schritte nicht aus. Wenn im Rahmen der vorliegenden Erfindung eine Zusammensetzung unter Verwendung des Ausdrucks "aufweisen" bzw. "aufweisend" beschrieben ist, schließt dies ausdrücklich Zusammensetzungen ein, die aus den genannten Bestandteilen bestehen oder im Wesentlichen aus den genannten Bestandteilen bestehen.

Der Ausdruck "bestehen aus" bzw. "bestehend aus" bezeichnet im Rahmen der vorliegenden Erfindung eine geschlossene Aufzählung und schließt neben den ausdrücklich genannten Bestandteilen bzw. Schritten jegliche anderen Bestandteile bzw. Schritte aus.

Der Ausdruck "im Wesentlichen bestehen aus" bzw. "im Wesentlichen bestehend aus" bezeichnet im Rahmen der vorliegenden Erfindung eine teilweise geschlossene Aufzählung und bezeichnet Zusammensetzungen, die neben den genannten Bestandteilen nur noch solche weitere Bestandteile aufweisen, die den Charakter der Zusammensetzung nicht materiell verändern oder die in Mengen vorliegen, die den Charakter der Zusammensetzung nicht materiell verändern.

Weitere Merkmale und Vorteile der Erfindung ergeben sich aus der nachfolgenden Beschreibung bevorzugter Ausführungsbeispiele.

**Beispiele**

Alle in den Beispielen vorgenommen Angaben in % beziehen sich, sofern nicht explizit anderweitig aufgeführt, auf Angaben in Gew.-%.

Beispiel 1: Herstellung eines Schafgarbe Frischpflanzenpresssaftkonzentrats durch Gefriertrocknung

100 ml Schafgarbe *(Achillea millefolium)* Frischpflanzenpresssaft (Firma Schoenenberger), hergestellt aus dem in Deutschland kultivierten, blühenden Kraut, werden eingefroren und anschließend gefriergetrocknet. 5,3 g Trockensaft 1a werden als braun-grüner Feststoff erhalten und die Zusammensetzung analysiert:

Tabelle 1:

| Substanzklasse | Gehalt [Gew.-%] |
|---|---|
| Gesamtpolyphenole (photometrisch, Folin-Denis-Methode als Catechin Äquivalente) | 16,4 |
| Beinhaltend | |
| Summe an Monocaffeoylchinasäuren (Chlorogensäure, Neo- und Cryptochlorogensäure, HPLC)* | 10,5 |
| Summe an Dicaffeoylchinasäuren (1,5-, 3,4-, 3,5-und 4,5-Dicaffeoylchinasäure, HPLC}* | 5,3 |
| Rutin (HPLC) | 0,3 |
| Gesamtproteine (Stickstoffanalysator) | 3,3 |
| Summe an Aminosäuren (HPLC) | 1,0 |
| Kalium $K^+$ (IC) | 10,2 |
| Calcium $Ca^{2+}$ (IC) | 0,7 |
| Magnesium $Mg^{2+}$ (IC) | 0,4 |
| Natrium $Na^+$ (IC) | 0,04 |
| Wasser (Karl Fischer) | 7,5 |
| * Quantifiziert als Chlorogensäure | |

Es werden von 3 weitere Chargen Schafgarbe (*Achillea millefolium*) Frischpflanzenpresssaft (Firma Schoenenberger) von jeweils 100 ml eingefroren und anschließend gefriergetrocknet. Die erhaltenen Trockensäfte 1b - 1d werden ausgewogen und auf Summe an Mono- und Dicaffeoylchinasäuren und Rutin analysiert:

Tabelle 2:

| Substanzklasse | 1a | 1b | 1b | 1d | Mittelwert |
|---|---|---|---|---|---|
| Ausbeute gefriergetrockneter Schafgarbe Frischpflanzenpresssaft | 5,3 | 4,5 | 4,9 | 4,1 | 4,7 ± 0,4 |
| Gehalt [Gew.-%] | | | | | |
| Summe an Monocaffeoylchinasäuren (Chlorogensäure., Neo- und Cryptochlorogensäure, HPLC)* | 10,5 | 8,9 | 11,4 | 7,7 | 9,6 ±1,3 |
| Summe an Dicaffeoylchinasäuren (1,5-, 3,4-, 3,5- und 4,5-Dicaffeoylchinasäure, HPLC)* | 5,3 | 3,9 | 5,7 | 3,1 | 4,5 ± 1,0 |
| Rutin (HPLC) | 0,3 | 0,4 | 0,3 | 0,1 | 0,3 ± 0,1 |
| Wasser (Karl Fischer) | 7,5 | n.b. | n.b. | 5,6 | 6,6 ± 1,0 |
| * Quantifiziert als Chlorogensäure<br>n.b. = nicht bestimmt | | | | | |

Die Ergebnisse zeigen, dass die 4 Chargen Schafgarbe *(Achillea millefolium)* Frischpflanzenpresssaft (Firma Schoenenberger) im Mittel 4,7 ± 0,4 Trockenrückstand enthalten.

Die gefriergetrockneten Schafgarbe Frischpflanzenpresssaftkonzentrate enthalten im Mittel nur noch 6,6 ± 1,0% Wasser, 9,6 ± 1,3% Summe an Monocaffeoylchinasäuren, 4,5 ± 1,0% Summe an Dicaffeoylchinasäuren und 0,3 ± 0,1% Rutin.

Beispiel 2: Herstellung eines Schafgarbe Frischpflanzenpresssaftkonzentrats durch Sprühtrocknung

1 l Schafgarbe Frischpflanzenpresssafts (Firma Schoenenberger, Trockensubstanzgehalt 5,3%) werden mit 150 g Maltodextrin DE 17-20 gemischt und sprühgetrocknet. Erhalten wird ein beiges, frei fließendes Pulver bestehend aus 75% Maltodextrin und 25% Schafgarbetrockenfrischpflanzenpresssaft. Das sprühgetrocknete Konzentrat wird auf Summe an Mono- und Dicaffeoylchinasäuren und Rutin analysiert:

Tabelle 3:

| Substanzklasse | Gehalt [Gew.-%] |
|---|---|
| Summe an Monocaffeoylchinasäuren (umfassend Chlorogensäure, Neo- und Cryptochlorogensäure, HPLC)* | 2,9 |
| Summe an Dicaffeoylchinasäuren (umfassend 1,5-, 3,4-, 3,5- und 3,4-Dicaffeoylchinasäure, HPLC)* | 1,4 |
| Rutin (HPLC) | 0,1 |
| Wasser (Karl Fischer) | 3,9 |
| * Quantifiziert als Chlorogensäure | |

Die analytischen Ergebnisse zeigen, dass die Inhaltsstoffe bei der Sprühtrocknung erhalten bleiben und kein Abbau stattfindet.

Beispiel 3: Effekt von Schafgarbe Frischpflanzenpresssaftkonzentrat auf die Genexpression von Hitzeschockproteinen in Hautzellen

Die neonatalen humanen epidermalen Keratinozyten (nHEKs) werden in Mikrotiterplatten mit 6 Vertiefungen mit einer Dichte von $0,2 \times 10^6$ Zellen/Vertiefung ausgesät und in EpiLife Medium kultiviert. Nach einer Inkubationszeit von 24 Stunden wird das Schafgarbe Frischpflanzenpresssaftkonzentrat aus Beispiel 1 in einer Konzentration von 0,005 Gew.-% in EpiLife aufgetragen und für 24 Stunden inkubiert.

Die Isolation und Aufreinigung der mRNA erfolgt mit Hilfe des RNeasy® Mini Kits der Firma Qiagen. Die Elution der mRNA von der Säule erfolgt mit 50μl RNase freiem Wasser. Die Quantifizierung und Bestimmung der Reinheit der isolierten RNA erfolgt spektrophotometrisch. Die Reinheit wird über die Verhältnisse der Extinktionen E260/E280 und E260/E230 beurteilt. Die Umschreibung der RNA in cDNA erfolgt nach Anleitung des Herstellers (RNA-to-cDNA Kit, Applied Biosystems). Die umgeschriebene Menge beträgt 0,5-1μg pro 6er-Vertiefung. Die umgeschriebenen Proben werden sofort in der PCR weiter verarbeitet oder bis zum Fortfahren des Experimentes bei -20 °C gelagert.

Tabelle 4: Umschreibungsbedingungen für die Reverse Transkription

| Vorgang | Dauer [min] | Temp. [C°] | Anzahl der Zyklen |
|---|---|---|---|
| Reverse Transkription | 60 | 37 | |
| Inaktivierung der Enzyme | 5 | 95 | |
| Halten einer Temperatur | bis das Gerät gestoppt wird | 4 | bis das Gerät gestoppt wird |

Im nächsten Schritt erfolgt die qRT-PCR. Für die qRT-PCR wird die cDNA aufgetaut und mit dem TaqMan® Fast Universal PCR Master Mix (2x) (Life Technologies) versetzt. Die TaqMan® Platte mit 96 Vertiefungen (Life Technologies, Darmstadt) kann individuell mit den gewünschten Primern ausgestattet werden. In jede Vertiefung der Arrayplatte mit 96 Vertiefungen wird ein Volumen von 10 μL Probe pipettiert und die Vertiefungen werden sorgfältig mit einem adhäsiven Film (MicroAmp®) luftdicht verschlossen und zentrifugiert. Die Genexpressionsanalyse erfolgt im StepOne Plus Fast Real-Time PCR Instrument (Applied Biosystems). Das Gerät durchläuft nach der Aufheizphase automatisch die in Tabelle 5 gezeigten Zyklen nacheinander.

Tabelle 5: Die eingestellten PCR-Zyklen

| Vorgang | Dauer (sec) | Temp. [C°] | Anzahl der Zyklen |
|---|---|---|---|
| Initiale cDNA Denaturierung | 20 | 95 | 1 |
| Denaturierung der cDNA | 3 | 95 | 40 |
| Annealing und Elongation | 30 | 60 | 40 |

Die gemessenen Werte werden in MS Office Excel exportiert und ausgewertet. Die Berechnung der relativen Genexpression erfolgt nach der ΔΔCT-Methode. Im ersten Schritt der Berechnung wird für jede Probe der ΔCT-Wert berechnet indem der CT-Wert des Referenzgens (Gen-HRPT) vom CT-Wert des zu untersuchenden Gens subtrahiert wird.

$$\Delta CTWert_{Zielgen} = CTWert_{Zielgen} - CTWert_{Referenzgen\ (HTRPGen)}$$

Nach der Standardisierung folgt die Subtraktion des ΔCT-Wertes der Kontrollprobe (unbehandelt) von dem ΔCT-Wert der behandelten Probe (PS aufgetragen).

$$\Delta\Delta CTWert_{Zielgen} = \Delta CTWert_{Behandelt} - \Delta CTWert_{Kontrolle}$$

Im letzten Schritt erfolgt die Berechnung des relativen Expressionsunterschiedes (relativer Quantifikations-Wert (RQ-Wert)) zwischen stimulierter Probe (PS aufgetragen) und Kontrolle.Relative Genexpression (RQ - Wert) = $2^{-\Delta\Delta CT}$

Ein RQ-Wert von >2,5 wird als relevante Induktion eines Gens betrachtet.

Tabelle 6: Ergebnisse

| Zelltyp | NHEK |
|---|---|
| Testkonzentration | 0,005% |
| RQ Werte | |
| HPRT1 (Housekeeping gen) | 1,0 |
| HSPB2 -> HSP27 | 8,0 |
| HSPA1L -> HSP70 | keine relevante Induktion |

Die Ergebnisse zeigen, dass Schafgarbe Frischpflanzenpresssaft die Genexpression von Hitzeschockprotein HSP27 bei sehr geringer Testkonzentration sehr effektiv hochreguliert, jedoch keinen Effekt auf HSP70 hat.

Beispiel 4: Effekt von Schafgarbe Frischpflanzenpresssaftkonzentrat auf die Genexpression von AMPs in Hautzellen

HaCaT Zellen werden in Mikrotiterplatten mit 6 Vertiefungen mit einer Dichte von $0,5 \times 10^6$ Zellen/Vertiefung ausgesät und in EpiLife Medium kultiviert.

Nach einer Inkubationszeit von 2 Tagen wird das Schafgarbe Frischpflanzenpresssaftkonzentrat aus Beispiel 1 in einer Konzentration von 0,005 Gew.-% in EpiLife aufgetragen und für 24 h inkubiert.

Die Isolation und Aufreinigung der mRNA erfolgt mit Hilfe des RNeasy® Mini Kits der Firma Qiagen. Die Elution der mRNA von der Säule erfolgt mit 50μl RNase freiem Wasser. Die Quantifizierung und Bestimmung der Reinheit der isolierten RNA erfolgt spektrophotometrisch. Die Reinheit wird über die Verhältnisse der Extinktionen E260/E280 und E260/E230 beurteilt.

Die Umschreibung der RNA in cDNA erfolgt nach Anleitung des Herstellers (RNA-to-cDNA Kit, Applied Biosystems). Die umgeschriebene Menge beträgt 0,5-1 μg pro 6er-Vertiefung. Die umgeschriebenen Proben werden sofort in der PCR weiter verarbeitet oder bis zum Fortfahren des Experimentes bei -20 °C gelagert.

Tabelle 7: Umschreibungsbedingungen für die Reverse Transkription

| Vorgang | Dauer [min] | Temp. [C°] | Anzahl der Zyklen |
|---|---|---|---|
| Reverse Transkription | 60 | 37 | |
| Inaktivierung der Enzyme | 5 | 95 | |
| Halten einer Temperatur | bis das Gerät gestoppt wird | 4 | bis das Gerät gestoppt wird |

Im nächsten Schritt erfolgt die qRT-PCR. Für die qRT-PCR wird die cDNA aufgetaut und mit dem TaqMan® Fast Universal PCR Master Mix (2x) (Life Technologies) versetzt. Die TaqMan® Platte mit 96 Vertiefungen (Life Technologies, Darmstadt) kann individuell mit den gewünschten Primern ausgestattet werden. In jede Vertiefung der Arrayplatte mit 96 Vertiefungen wird ein Volumen von 10 μL Probe pipettiert und die Vertiefungen werden sorgfältig mit einem adhäsiven Film (MicroAmp®) luftdicht verschlossen und erneut eine Minute zentrifugiert. Die Genexpressionsanalyse erfolgt im StepOne Plus Fast Real-Time PCR Instrument (Applied Biosystems). Das Gerät durchläuft nach der Aufheizphase automatisch die in Tabelle 8 gezeigten Zyklen nacheinander.

Tabelle 8: Die eingestellten PCR-Zyklen

| Vorgang | Dauer [sec] | Temp. [C°] | Anzahl der Zyklen |
|---|---|---|---|
| Initiale cDNA Denaturierung | 20 | 95 | 1 |
| Denaturierung der cDNA | 3 | 95 | 40 |
| Annealing und Elongation | 30 | 60 | 40 |

Die gemessenen Werte werden exportiert und mittels MS Office Excel ausgewertet. Die Berechnung der relativen Genexpression erfolgt nach der ΔΔCT-Methode. Im ersten Schritt der Berechnung wird für jede Probe der ΔCT-Wert berechnet indem der CT-Wert des Referenzgens (Gen-HRPT) vom CT-Wert des zu untersuchenden Gens subtrahiert wird.

$$\Delta CTWert_{Zielgen} = CTWert_{Zielgen} - CTWert_{Referenzgen\ (HTRPGen)}$$

Nach der Standardisierung folgt die Subtraktion des ΔCT-Wertes der Kontrollprobe (unbehandelt) von dem ΔCT-Wert der behandelten Probe (PS aufgetragen).

$$\Delta\Delta CTWert_{Zielgen} = \Delta CTWert_{Behandelt} - \Delta CTWert_{Kontrolle}$$

Im letzten Schritt erfolgt die Berechnung des relativen Expressionsunterschiedes (relativer Quantifikations-Wert (RQ-Wert)) zwischen stimulierter Probe (PS aufgetragen) und Kontrolle.

Relative Genexpression (RQ - Wert) = $2^{-\Delta\Delta CT}$

Ein RQ-Wert von >2,5 wird als relevante Induktion eines Gens betrachtet.

Tabelle 9: Ergebnisse

| Zelltyp | HaCaT |
|---|---|
| Testkonzentration | 0,005% |
| RQ Werte | |
| HPRT1 (Housekeeping gen) | 1,0 |
| DEFB1 -> beta-Defensin 1 | 9,1 |
| S100A8 -> Calgranulin A | 2,7 |

Die Ergebnisse zeigen, dass Schafgarbe Frischpflanzenpresssaft die Genexpression der antimikrobiellen Peptide beta-Defensin 1 und S100 Calcium Bindeprotein A8 bei sehr geringer Testkonzentration sehr effektiv hochreguliert.

Beispiel 5: Formulierungsbeispiele

1 = Beruhigungsbalsam für die Haut

2 = Getönter Anti-Aging Balmsam, SPF 15

3 = After-sun Feuchtigkeitsspray O/W

4 = Nachtcreme W/O

5 = Gesichtsreinigungsgel

6 = After-Shave Hydrogel

7 = Antischuppen Haarshampoo

8 = Schweißhemmendes Pumpspray

9 = Tagespflegefluid zur Hautaufhellung O/W

10 = Creme zur Wiederherstellung der Barrierefunktion O/W

11 = Sonnenschutzlotion SPF 24 (UVA/UVB Balance)

Bei den Formulierungsbeispielen 1 - 11 werden die nachstehend beschriebenen zwei Parfümöle PFO1 und PFO2 jeweils als Duftstoff verwendet (DPG = Dipropylenglycol).

Tabelle 10: Parfüm öl PFO1 mit Rosenduft

| Bestandteil | Menge (in Gew.-%) |
|---|---|
| Acetophenon, 10% in DPG | 10 |
| n-Undecanal | 5 |
| Aldehyd C14 (sogenannter Pfirsichaldehyd) | 15 |
| Allylamylglycolat, 10% in DPG | 20 |
| Amylsalicylat | 25 |
| Benzylacetat | 60 |
| Citronellol | 80 |
| d-Limonen | 50 |
| trans-9 Decenol | 15 |
| Dihydromyrcenol | 50 |
| Dimethylbenzylcarbinylacetat | 30 |
| Diphenyloxid | 5 |
| Eucalyptol | 10 |
| Geraniol | 40 |
| Nerol | 20 |
| Geranienöl | 15 |

(fortgesetzt)

| Bestandteil | Menge (in Gew.-%) |
|---|---|
| cis-3 Hexenol, 10% in DPG | 5 |
| cis-3 Hexenylsalicylat | 20 |
| Indol, 10% in DPG | 10 |
| Alpha-Ionon | 15 |
| Beta-Ionon | 5 |
| Lilial® (2-Methyl-3-(4-tert-butyl-phenyl)propanal) | 60 |
| Linalool | 40 |
| Methylphenylacetat | 10 |
| Phenylethylalcohol | 275 |
| Styrolylacetat | 20 |
| Terpineol | 30 |
| Tetrahydrolinalool | 50 |
| Cinnamylalkohol | 10 |
| Insgesamt | 1000 |

Tabelle 11: Parfümöl PFO2 mit weißem Blüten und Moschusduft

| Bestandteil | Menge (in Gew.-%) |
|---|---|
| Benzylacetat | 60 |
| Citronellylacetat | 60 |
| Cyclamenaldehyd (2-Methyl-3-(4-isopropylphenyl)propanal | 20 |
| Dipropylenglycol (DPG) | 60 |
| Ethyllinalool | 40 |
| Florol (2-Isobutyl-4-methyltetrahydro-2H-pyran-4-ol) | 30 |
| Globanone® [(E/Z)-8-Cyclohexadecen-1-on] | 180 |
| Hedione® (Methyldihydrojasmonat) | 140 |
| Hexenylsalicylat, cis-3 | 10 |
| Vertocitral (2,4-Dimethyl-3-cyclohexenecarboxaldehyd) | 5 |
| Hydratropaaldehyd, 10% in DPG | 5 |
| Isodamascon (1-(2,4,4-Trimethyl-2-cyclohexen-1-yl)-2-buten-1-on), 10% in DPG | 5 |
| Isomuscon (Cyclohexadecanon) | 40 |
| Jacinthaflor (2-Methyl-4-phenyl-1,3-dioxolan) | 10 |
| Cis-jasmon, 10% in DPG | 20 |
| Linalool | 50 |
| Linalylacetat | 30 |
| Methylbenzoat, 10% in DPG | 25 |
| para-Methylcresol, 10% in DPG | 10 |
| Nerol | 20 |

(fortgesetzt)

| Bestandteil | Menge (in Gew.-%) |
|---|---|
| Phenylpropylaldehyd | 5 |
| 2-Phenylethylalkohol | 82 |
| Tetrahydrogeraniol | 13 |
| 2,2-Dimethyl-3-cyclohexyl-1-propanol | 80 |
| Insgesamt: | 1000 |

Tabelle 12: Kosmetische Formulierungen (Mengen in Gew.-%)

| Bestandteile | 1 | 2 | 3 | 4 | 5 | 6 | 7 | 8 | 9 | 10 | 11 |
|---|---|---|---|---|---|---|---|---|---|---|---|
| **Sprühgetrocknetes Schafgarbe Frischpflanzenpresssaft Konzentrat enthaltend 75% Maltodextrin**<br>Maltodextrin, Achillea millefolium extract | | 1.2 | | | 0.5 | 0.1 | | | 0.3 | | 0.02 |
| **Schafgarbe Frischpflanzenpresssaft Konzentrat, 10% Trockenrückstand**<br>Achillea millefolium extract, Glycerin | | | 2.5 | | | | 1 | 0.02 | | | |
| **Gefriergetrocknetes Schafgarbe Frischpflanzenpresssaft Konzentrat**<br>Achillea millefolium extract | 0.01 | | | 0.002 | | | | | | 0.05 | |
| **Actipone® Laminaria Saccharina**<br>Glycerin, Water (Aqua), Laminaria Saccharina Extract | | | | | | 0.3 | | | | | |
| **Allantoin**<br>Allantoin | 0.1 | | | | | 0.1 | | | | | |
| **Aloe Vera Gel Conc.10:1**<br>Aloe Barbadensis(Aloe) Leaf Juice | | | | | | 1 | | | | | |
| **Aluminium Stearat**<br>Aluminium Stearate | | | | 1.2 | | | | | | | |
| **Beta-Arbutin**<br>Arbutin | | | | | | | | | 1 | | |
| **Arlypon® F**<br>Laureth-2 | | | | | | | 2 | | | | |
| **Avocado Öl**<br>Persea Gratissima (Avocado) Oil | | | 3 | | | | | | | | |
| **Betulin 90%**<br>Betulin | | | | | | | | | | 0.1 | |
| **Biotive L-Arginin**<br>Arginine | | 0.6 | | | | | | | | | 0.5 |
| **Biotive Troxerutin**<br>Troxerutin | | 0.5 | | | | | | | | | 0.5 |
| **(-)-alpha-Bisabolol**<br>Bisabolol | | | | | | | | | | | 0.1 |

(fortgesetzt)

| Bestandteile | 1 | 2 | 3 | 4 | 5 | 6 | 7 | 8 | 9 | 10 | 11 |
|---|---|---|---|---|---|---|---|---|---|---|---|
| **Carbopol Aqua SF-1 Polymer** Acrylates Copolymer | | | | | 5 | | | | | | |
| **Carbopol® Ultrez-10** Carbomer | | 0.2 | | | | 0.4 | | | 0.2 | | |
| **CeramideBIO®** Cetylhydroxyproline Palmitamide | | | | | | | | | | 0.5 | |
| **Citronensäure 10% in Wasser** | | | | | 0.2 | | 0.5 | | | | |
| **Covi-Ox® T-70** Tocopherol | | | 0.1 | | | | | | | | |
| **Crinipan® AD** Climbazole | | | | | | | 0.3 | | | | |
| **Cutina® PES** Pentaerythrityl Distearate | | 2 | | | | | | | | | |
| **D-Panthenol** Panthenol | 1 | | 1 | | | 0.5 | 0.5 | | | | |
| **Dehyton K** Cocamidopropyl Betaine | | | | | 8 | | 8 | | | | |
| **Dermacryl® AQF Acrylates** Copolymer | | | | | | | | | | | 2 |
| **Dow Corning 200(100cs)** Silicone Fluid Dimethicone | 2 | 2 | | | | | | | 0.5 | | |
| **Dow Corning 246 Fluid** Cyclohexasiloxane, Cyclopentasiloxane | | | 2 | | | | | | | | 3 |
| **Dracorin® CE** Glyceryl Stearate Citrate | | | | | | | | | | 1.5 | |
| **Dracorin® GMS** Glyceryl Stearate | | | | | | | | | | 2 | |

23

| Bestandteile | 1 | 2 | 3 | 4 | 5 | 6 | 7 | 8 | 9 | 10 | 11 |
|---|---|---|---|---|---|---|---|---|---|---|---|
| **Dracorin® GOC**<br>lyceryl Oleate Citrate, Caprylic/Capric Triglyceride | | | 2 | | | | | | | | |
| **Dragocalm®**<br>Water (Aqua), Glycerin, Avena Sativa (Oat) Kernel Extract | 1 | | | | | | | | | | |
| **Dragocid® Liquid**<br>Phenoxyethanol, Methylparaben, Ethylparaben, Butylparaben, Propylparaben, Isobutylparaben | | | | | | | | | 0.8 | | |
| **Dragoderm®**<br>Glycerin, Triticum Vulgare (Wheat) Gluten, Water(Aqua) | | | | | | | 0.5 | | | | |
| **Dragosan® W/O P**<br>Sorbitan Isostearate, Hydrogenated Castor Oil, Ceresin, Beeswax (Cera Alba) | | | | 8 | | | | | | | |
| **Dragosantol® 100**<br>Bisabolol | | | | | 0.2 | | | | | | |
| **Dragosine®**<br>Carnosine | | 0.2 | | | | | | | | | |
| **Dragoxat® 89**<br>Ethylhexyl Isononanoate | | 5 | | 7 | | | | | | 2 | 2 |
| **Dinatrium EDTA** | 0.1 | 0.1 | | | | | | | 0.1 | | 0.1 |
| **Emulsiphos®**<br>Potassium Cetyl Phosphate, Hydrogenated Palm Glycerides | | 2 | | | | | | | 1.5 | 2 | 2 |
| Ethanol | | | 5 | | | 8 | | | | | |
| **Extrapone® Aloe vera**<br>Water (Aqua), Aloe Barbadensis, Propylene Glycol, Alcohol | | | | | | | 2 | | | | |
| **Extrapone® Witch Hazel**<br>Propylene Glycol, Hamamelis Virginiana (Witch Hazel) Water, Water (Aqua), Hamamelis Virginiana (Witch Hazel) Extract | 1 | | | | | | | | | | |
| **Extrapone® Rosemary**<br>Glycerin, Water (Aqua), | | | | | | | 0.3 | | | | |

EP 3 615 147 B1

| Bestandteile | 1 | 2 | 3 | 4 | 5 | 6 | 7 | 8 | 9 | 10 | 11 |
|---|---|---|---|---|---|---|---|---|---|---|---|
| Rosmarinus Officinalis (Rosemary) Leaf Extract | | | | | | | | | | | |
| **Extrapone® Seaweed Water** (Aqua), Butylene Glycol, Fucus Vesiculosus Extract | | | | | | 0.5 | | | | | |
| **Farnesol DT** Phenoxyethanol, Farnesol, Bisabolol | | | | | | | | 0.2 | | | |
| **Lebensmittelfarbstoff Braun** E172+E171 Powder | | 2 | | | | | | | | | |
| **Frescolat® MGA** Menthone Glycerin Acetal | | | 0.1 | | | | | | | | |
| **Frescolat® ML** Menthyl Lactate | | | 0.5 | | | 0.3 | 0.2 | | | | |
| **Frescolat® X-Cool** Menthyl Ethylamido Oxalate | | | | | | | | | | 0.2 | |
| **Genapol® LRO Liquid** Sodium Laureth Sulfate | | | | | | | 37 | | | | |
| **Givobio® GZN** Zinc Gluconate | | | | | | | | | | 0.5 | |
| **Glycerin** | 1.5 | | 4 | 3 | | | | | 3.5 | 3 | 3 |
| **Hydrolite® 5** Pentylene Glycol | 3 | | 5 | | 2 | 5 | | 5 | | | 2 |
| **Hydroviton-24®** Water (Aqua), Pentylene Glycol, Glycerin, Lactic Acid, Sodium Lactate, Serine, Urea, Sorbitol, Sodium Chloride, Allantoin | | | | 1 | | | | | | | |
| **Hydroviton® Plus 2290** Water (Aqua), Pentylene Glycol, Glycerin, Fructose, Urea, Citric acid, Sodium Hydroxide, Maltose, Sodium PCA, Sodium Chloride, Sodium Lactate, Trehalose, Allantoin, Sodium Hyaluronate, Glucose | | | | | | | | 1 | | 2 | |
| **Isoadipate** Diisopropyl Adipate | | | | | | | | | 2 | | |

EP 3 615 147 B1

(fortgesetzt)

| Bestandteile | 1 | 2 | 3 | 4 | 5 | 6 | 7 | 8 | 9 | 10 | 11 |
|---|---|---|---|---|---|---|---|---|---|---|---|
| **Isodragol®** Triisononanoin | 1 | | | | | | | | | 3 | 2 |
| **Jojoba Öl** Simmondsia Chinensis (Jojoba) Seed Oil | | | | 2 | | | | | | | |
| **Kaliumsorbat** | | | 0.1 | | | | | | | | |
| **Keltrol® CG-RD** Xanthan Gum | | 0.2 | | | | | | | 0.2 | | 0.4 |
| **Kojisäure** Kojic acid | | | | | | | | | 0.5 | | |
| **Lanette® 16** Cetyl Alcohol | | | | | | | | | 1.5 | | 1 |
| **Lanette® O** Cetearyl Alcohol | | | | | | | | | | 2 | 0.5 |
| **Lara Care® A-200** Galactoarabinan | | | | | | | | | | | 0.3 |
| **Locron® L** Aluminium Chlorohydrate | | | | | | | | 16 | | | |
| **Magnesiumsulfat** | | | | 0.7 | | | | | | | |
| **Mineralöl** | | | | 8 | | | | | | | |
| **Natriumascorbylphosphat** | | | | | | | | | 1 | | |
| **Natriumchlorid** | | | | | | | 0.1 | | | | |
| **Natriumhydroxid 10% in Wasser** | 1 | | | | 2 | 0.7 | | | | | |
| **Neo Heliopan® 303** Octocrylene | | 4 | | | | | | | 0.2 | 0.3 | 10 |
| **Neo Heliopan® 357** Butylmethoxydibenzoylmethane | | 2 | | | | | | | 2 | | 3 |

| Bestandteile | 1 | 2 | 3 | 4 | 5 | 6 | 7 | 8 | 9 | 10 | 11 |
|---|---|---|---|---|---|---|---|---|---|---|---|
| **Neo Heliopan® AP,** 15% Lösung, neutralisiert mit L-Arginin Aqua, Disodium Phenyl Dibenzimidazole Tetrasulfonate, Arginin | | 6.7 | | | | | | | | | 6.7 |
| **Neo Heliopan® AV** Ethylhexyl Methoxycinnamate | | | | | | | | | 7.5 | | |
| **Neo Heliopan® BB** Benzophenone-3 | | | | | | | | | 3 | | |
| **Neo Heliopan® E 1000** Isoamyl p.Methoxycinnamate | | | | | | | | | | | 1 |
| **Neo Heliopan® HMS** Homosalate | | | | | | | | | 10 | | 5 |
| **Neo Heliopan® OS** Ethylhexyl Salicylate | | 3 | | | | | | | 5 | | |
| **Neo Heliopan® Hydro,** 20% Lösung, neutralisiert mit Biotive Arginine Aqua, Phenylbenzimidazole, Sulphonic Acid, Arginin | | 10 | | | | | | | | | 10 |
| **Neo-PCL Water Soluble N** Trideceth-9, PEG-5 Ethylhexanoate, Water (Aqua) | | | | | | 1 | 1.5 | 2 | | | |
| **Neutralöl** Caprylic/Capric Triglyceride | | | 5 | | | | | | | 10 | |
| **Niacinamid** | | | | | 1 | | | | | | |
| **Ozokerite Wax 2389** Ozokerite | | | | 2 | | | | | | | |
| **Parfumöl PFO1 or PFO2** Parfum | 0.05 | 0.3 | 0.25 | 0.3 | | 0.1 | 0.5 | 0.7 | 0.3 | 0.1 | 0.2 |
| **PCL-Liquid 100** Cetearyl Ethylhexanoate | 3 | 2 | 4 | 5 | | | | | | | |

| Bestandteile | 1 | 2 | 3 | 4 | 5 | 6 | 7 | 8 | 9 | 10 | 11 |
|---|---|---|---|---|---|---|---|---|---|---|---|
| **PCL-Solid** Stearyl Heptanoate, Stearyl Caprylate | 1 | | 0.5 | | | | | | | | |
| **Pemulen® TR-2** Acrylates/C10-30 Alkyl Acrylate Crosspolymer | 0.6 | | 0.25 | | | | | | | | |
| **Phenethylalkohol** | | | | | 0.2 | | | | | | |
| **Phenoxyethanol** | | | | | | | | | | | 0.2 |
| **Phytoconcentrole®** Shea Butter Glycine Soja (Soybean) Oil, Butyrospermum Parkii (Shea Butter) | 1 | | | | | | | | | | |
| **Polymer JR 400** Polyquaternium-10 | | | | | | | 0.4 | | | | |
| **Propylenglykol-1,2** Propylene Glycol | | | | | | 5 | | 3 | | | |
| **Silcare Silicone 41M65** Stearyl Dimethicone | | | | | | | | | | | 1 |
| **Solubilizer** PEG-40 Hydrogenated Castor Oil, Trideceth-9, Propylene Glycol, Water (Aqua) | | | | | | | | 3 | | | |
| **Sulfetal LA** Ammonium Lauryl Sulfate | | | | | 12 | | | | | | |
| **SymCalmin®** Butylene Glycol, Pentylene Glycol, Hydroxyphenyl Propamidobenzoic Acid | 1 | | | | | | 0.1 | | 0.1 | | |
| **SymClariol®** Decylene Glycol | | 0.5 | | | 1 | | | | 0.3 | | |
| **SymDecanox HA** Caprylic/Capric Triglyceride, Hydroxymethoxyphenyl Decanone | | | 2 | | | | | | | | |
| **SymDeo® B125** 2-Methyl 5-Cyclohexylpentanol | | | | | | | 0.2 | | | | |

EP 3 615 147 B1

(fortgesetzt)

| Bestandteile | 1 | 2 | 3 | 4 | 5 | 6 | 7 | 8 | 9 | 10 | 11 |
|---|---|---|---|---|---|---|---|---|---|---|---|
| **SymDeo® MPP** <br> Dimethyl Phenyl 2-Butanol | | | | | | | | 0.5 | | | |
| **Symdiol® 68** <br> 1.2-Hexanediol, Caprylyl Glycol | 1 | 0.5 | | | | | | | | | 0.3 |
| **SymFinity® 1298** <br> Echinacea Purpurea Extract | | | | 0.05 | | | | | | | |
| **SymGlucan®** <br> Water (Aqua), Glycerin, Beta-Glucan | 1 | | 5 | | | | | | | | 2 |
| **SymHair® Force 1631** <br> Pentylene Glycol, Isochrysis galbana Extract | | | | | | | 2 | | | | |
| **SymHelios® 1031** <br> Benzylidene Dimethoxydimethylindanon e | | 0.5 | | | | | | | | | |
| **SymLift** <br> Water, trehalose, glycerin, pentylene glycol, beta-glucan, hordeum vulgare seed extract, sodium hyaluronate, 1,2-Hexanediol, caprylyl glycol, sodium benzoate, maltodextrine | | 2 | | | | | | | | | |
| **SymMatrix** <br> Maltodextrin, Rubus Fruticosus (Blackberry) Leaf Extract | | 0.1 | 0.3 | | | | | | | | |
| **SymMollient® W/S** <br> Trideceth-9, PEG-5 Isononanoate, Water (Aqua) | | | | | 2 | | 2 | | | | |
| **SymOcide® C** <br> o-Cymen-5-ol | | | | | | 0.1 | | | | | |
| **SymOcide® PH** <br> Phenoxyethanol, Hydroxyacetophenone, Caprylyl Glycol, Water (Aqua) | | | | | | | 1.0 | | | | |
| **SymOcide® PS** <br> Phenoxyethanol, Decylene | | | | | | | | | | 0.8 | |
| Glycol, 1,2-Hexanediol | | | | | | | | | | | |

(fortgesetzt)

| Bestandteile | 1 | 2 | 3 | 4 | 5 | 6 | 7 | 8 | 9 | 10 | 11 |
|---|---|---|---|---|---|---|---|---|---|---|---|
| **SymOcide® PT**<br>Phenoxyethanol, Tropolone | | | | 0.8 | | | | | | | |
| **SymPeptide® 225**<br>Glycerin, Water (Aqua), Myristoyl Pentapeptide-11 | | | | 1 | | | | | | | |
| **SymRelief® 100**<br>Bisabolol, Zingiber Officinale (Ginger) Root Extract | | | | | | 0.2 | | | | | |
| **SymRelief S**<br>Bisabolol, Hydroxymethoxyphenyl Decanone | | | | | | | | | | 0.1 | |
| **SymRepair® 100**<br>Hexyldecanol, Bisabolol, Cetylhydroxyproline Palmitamide, Stearic Acid, Brassica Campestris (Rapeseed) Sterols | | 1 | | 3 | | | | | | | |
| **SymSave® H**<br>Hydoxyacetophenone | | 0.5 | | | 0.8 | 0.5 | | | | | 0.5 |
| **SymSol® PF-3**<br>Water (Aqua), Pentylene Glycol, Sodium Lauryl Sulfoacetate, Sodium Oleoyl Sarcosinate, Sodium Chloride, Disodium Sulfoacetate, Sodium Oleate, Sodium Sulfate | | | | | | 1.3 | | | | | |
| **SymSitive® 1609**<br>Pentylene Glycol, 4-t-Butylcyclohexanol | | | 0.5 | | | | | | 0.5 | | |
| **SymVital® AgeRepair 3040**<br>Zingiber Officinale (Ginger) Root Extract | | | 0.1 | | | | | | | | |
| **SymWhite® 377**<br>Phenylethyl Resorcinol | | | | | | | | | 0.5 | | |
| **Tamasterol®**<br>Phytosterols | | | | | | | | | | 0.3 | |
| **Tapioca Pure**<br>Tapioca Starch | | | | | | | | | | | 5 |

EP 3 615 147 B1

(fortgesetzt)

| Bestandteile | 1 | 2 | 3 | 4 | 5 | 6 | 7 | 8 | 9 | 10 | 11 |
|---|---|---|---|---|---|---|---|---|---|---|---|
| **Tegosoft® PC 31** <br> Polyglyceryl-3 Caprate | | | | | | | | | | 0.3 | |
| **Triethanolamin** | | | 0.3 | | | | | | | | |
| **Vitamin A Palmitat** <br> Retinyl Palmitate | | | | 0.1 | | | | | | | |
| **Vitamin E Acetat** <br> Tocopheryl Acetate | | 0.5 | | 0.2 | | | | | | 0.3 | 0.5 |
| **Zetesol LA-2** <br> Ammonium Laureth Sulfate | | | | | 26 | | | | | | |
| **Wasser** | Ad 100 |||||||||||

Tabelle 13: Gelzahncreme

| Bestandteil | I (%) | II (%) | III (%) |
|---|---|---|---|
| Natriumcarboxymethylcellulose | 0.40 | 0.40 | 0.40 |
| Sorbitol 70 %, in Wasser | 72.00 | 72.00 | 72.00 |
| Polyethylenglycol (PEG) 1500 | 3.00 | 3.00 | 3.00 |
| Natriumsaccharinat | 0.07 | 0.07 | 0.07 |
| Natriumfluorid | 0.24 | 0.24 | 0.24 |
| p-Hydroxybenzoesäure (PHB) ethylester | 0.15 | 0.15 | |
| SymDiol 68 | | | 0.5 |
| SymSave H | | | 0.25 |
| Pfefferminzaroma | 1.00 | 1.00 | 1.00 |
| Sprühgetrocknetes Schafgarbe Frischpflanzen-presssaft Konzentrat enthaltend 75% Maltodextrin | 0.5 | | 0.1 |
| Schafgarbe Frischpflanzenpresssaft Konzentrat, 10% Trockenrückstand in Glycerin / Wasser | | 1 | |
| Abrasives Silica | 11.00 | 11.00 | 11.00 |
| Verdickendes Silica | 6.00 | 6.00 | 6.00 |
| Natriumdodecylsulfat (SDS) | 1.40 | 1.40 | 1.40 |
| Dest. Wasser | ad 100.00 | ad 100.00 | ad 100.00 |

Tabelle 14: Gebrauchsfertige Mundspülung mit Fluorid

| Bestandteil | I (%) | II (%) | III (%) |
|---|---|---|---|
| Ethanol | 7.00 | 7.00 | |
| Glycerin | 12.00 | 12.00 | |
| Natriumfluorid | 0.05 | 0.05 | 0.18 |
| Pluronic F-127® (BASF, oberflächenaktive Substanz) | 1.40 | 1.40 | |
| Natriumphosphatpuffer pH 7.0 | 1.10 | 1.10 | |
| Sorbinsäure | 0.20 | 0.20 | |
| Natriumsaccharinat | 0.10 | 0.10 | 0.10 |
| Zimt/Menthol Aroma | 0.15 | 0.15 | 0.15 |
| Sprühgetrocknetes Schafgarbe Frischpflanzen-presssaft Konzentrat enthaltend 75% Maltodextrin | 0.05 | | 0.8 |
| Schafgarbe Frischpflanzenpresssaft Konzentrat, 10% Trockenrückstand in Glycerin / Wasser | | 0.5 | |
| Farbstoff | 0.01 | 0.01 | 0.01 |
| Sorbitol 70% | | | 10 |
| Cremophor RH455 | | | 1.8 |
| SymDiol 68 | | | 0.5 |
| SymSave H | | | 0.2 |
| Dest. Wasser | ad 100.00 | ad 100.00 | ad 100.00 |

**Patentansprüche**

1. Festes Schafgarbe Frischpflanzenpresssaft Konzentrat, umfassend oder bestehend aus:

   10-50 Gew.-% Schafgarbe Frischpflanzenpresssaft Trockenrückstand,
   0-10 Gew.-% Wasser, und
   50-90 Gew.-% Trägerstoff(e),
   bezogen auf 100 Gew.-% Schafgarbe Frischpflanzenpresssaft Konzentrat,
   wobei der bzw. die Trägerstoff(e) ein fester Trägerstoff, ausgewählt aus der Gruppe bestehend aus Maltodextrin, Dextrin und Cyclodextrin, ist bzw. sind,
   wobei das Scharfgarbe Frischpflanzenpresssaft Konzentrat eine oder mehrere Dicaffeoylchinasäure(n) enthält,
   wobei eine oder mehrere oder alle Dicaffeoylchinasäure(n) ausgewählt ist/sind aus der Gruppe bestehend aus 3,4-Dicaffeoylchinasäure, 3,5-Dicaffeoylchinasäure und 4,5-Dicaffeoylchinasäure.

2. Schafgarbe Frischpflanzenpresssaft Konzentrat gemäß Anspruch 1, wobei das Schafgarbe Frischpflanzenpresssaft Konzentrat weiterhin Konservierungsmittel und/oder Stabilisatoren umfasst.

3. Schafgarbe Frischpflanzenpresssaft Konzentrat gemäß Anspruch 1 oder 2, wobei das Schafgarbe Frischpflanzenpresssaft Konzentrat hergestellt oder herstellbar ist durch ein Verfahren, das Sprühtrocknung umfasst.

4. Verfahren zur Herstellung eines Schafgarben Frischpflanzenpresssaft Konzentrats nach einem der vorangehenden Ansprüche, umfassend die Schritte oder bestehend aus den Schritten:

   Bereitstellen eines Schafgarben Frischpflanzenpresssafts, und
   Konzentrieren des Schafgarben Frischpflanzenpresssafts.

5. Verfahren gemäß Anspruch 4, wobei das Konzentrieren des Schafgarben Frischpflanzenpresssafts bei einer Temperatur von < 60°C erfolgt.

6. Kosmetische Zusammensetzung umfassend Schafgarbe Frischpflanzenpresssaft Konzentrat gemäß einem der Ansprüche 1 bis 3.

7. Pharmazeutische Zusammensetzung, Nahrungsmittelzusammensetzung oder Nahrungsergänzungsmittel, umfassend Schafgarbe Frischpflanzenpresssaft Konzentrat gemäß einem der Ansprüche 1 bis 3.

8. Kosmetische Zusammensetzung gemäß Anspruch 6 oder pharmazeutische Zusammensetzung gemäß Anspruch 7, weiter umfassend 0,01-10 Gew.-% eines hautberuhigenden Mittels und/oder eines Antioxidans.

9. Kosmetische, nicht-therapeutische Verwendung von Schafgarbe zur Stimulierung der Expression von Hitzeschockproteinen (HSP) in Hautzellen.

10. Kosmetische, nicht-therapeutische Verwendung von Schafgarbe zur Erhöhung der Thermotoleranz der Haut.

11. Verwendung von Schafgarbe nach einem der Ansprüche 9 oder 10, wobei die Schafgarbe als Schafgarbe Frischpflanzenpresssaft oder als kosmetische Zusammensetzung gemäß einem der Ansprüche 6 oder 8 vorliegt.

12. Verwendung von Schafgarbe nach Anspruch 11, wobei die Dosierung von Schafgarbe Frischpflanzenpresssaft Konzentrat in der kosmetischen Zusammensetzung 0.0001 - 10 Gew.-% bezogen auf 100 Gew.-% der kosmetischen Zusammensetzung beträgt.

13. Pharmazeutische Zusammensetzung gemäß Anspruch 7 zur Verwendung als Medikament.

14. Verwendung von Schafgarbe gemäß einem der Ansprüche 9 bis 12, wobei eine Anwendung topisch auf die Haut und/oder die Haare erfolgt.

15. Pharmazeutische Zusammensetzung zur Verwendung als Medikament gemäß Anspruch 13, wobei eine Anwendung topisch auf die Haut und/oder die Haare erfolgt.

**Claims**

1.  Solid yarrow fresh plant press juice concentrate comprising or consisting of:

    10 - 50 wt.-% yarrow fresh plant press juice dry residue,
    0-10 wt.-% water, and
    50 - 90 wt.-% carrier(s),
    based on 100 wt.-% yarrow press plant press juice concentrate,
    wherein the carrier(s) is/are a solid carrier, selected from the group consisting of maltodextrin, dextrin and cyclodextrin,
    wherein the yarrow fresh plant press juice concentrate comprises of one ore more dicaffeoylquinic acid(s),
    wherein one or more or all dicaffeoylquinic acid(s) are/is selected from the group consisting of 3,4-dicaffeoylquinic acid, 3,5-dicaffeoylquinic acid and 4,5-dicaffeoylquinic acid.

2.  Yarrow fresh plant press juice concentrate according to claim 1, wherein the yarrow fresh plant press juice concentrate further comprises preservatives and/or stabilizers.

3.  Yarrow fresh plant press juice concentrate according to claim 1 or 2, wherein the yarrow fresh plant press juice concentrate is produced or producible by a method comprising spray drying.

4.  Method for producing a yarrow fresh plant press juice concentrate according to any of the preceding claims, comprising the steps or consisting of the steps:

    providing a yarrow fresh plant press juice, and
    concentrating the yarrow fresh plant press juice.

5.  Method according to claim 4, wherein the concentrating of the yarrow fresh plant press juice takes place at a temperature of < 60°C.

6.  Cosmetic composition comprising yarrow fresh plant press juice concentrate according to any of claims 1 to 3.

7.  Pharmaceutical composition, food composition or food supplement comprising yarrow fresh plant press juice concentrate according to any of claims 1 to 3.

8.  Cosmetic composition according to claim 6 or pharmaceutical composition according to claim 7, further comprising 0.01 - 10 wt.-% of a skin soothing agent and/or an antioxidant.

9.  Cosmetic, non-therapeutic use of yarrow for stimulating the expression of heat shock proteins (HSP) in skin cells.

10. Cosmetic, non-therapeutic use of yarrow for increasing the thermal tolerance of the skin.

11. Use of yarrow according any of the claims 9 or 10, wherein the yarrow is present as yarrow fresh plant press juice or as a cosmetic composition according to any of claims 6 or 8.

12. Use of yarrow according to claim 11, wherein the dosage of yarrow fresh plant press juice concentrate in the cosmetic composition is 0.0001 - 10 wt.-% based on 100 wt.-% of the cosmetic composition.

13. Pharmaceutical composition according to claim 7 for use as a medicament.

14. Use of yarrow according to any of claims 9 to 12, wherein an application takes place topically on skin and/or hair.

15. Pharmaceutical composition for use as a medicament according to claim 13, wherein an application takes place topically on skin and/or hair.

**Revendications**

1.  Concentré de jus pressé de plante fraîche d'achillée millefeuille, comprenant ou consistant en:

10 à 50 % en poids de résidus secs de jus pressé de plante fraîche d'achillée millefeuille,

0 à 10 % en poids d'eau et

50 à 90 % en poids de matériau(x) support(s),

par rapport à 100 % en poids de concentré de jus pressé de plante fraîche d'achillée millefeuille,

dans lequel le ou bien les matériau(x) support(s) est ou bien sont un matériau support solide choisi dans le groupe constitué de la maltodextrine, de la dextrine et de la cyclodextrine,

dans lequel le concentré de jus pressé de plante fraîche d'achillée millefeuille contient un ou plusieurs acide(s) dicaféoylquinique(s), dans lequel un ou plusieurs ou tous les acides dicaféoylquiniques est ou bien sont choisi(s) dans le groupe constitué de l'acide 3,4-dicaféoylquinique, de l'acide 3,5-dicaféoylquinique et de l'acide 4,5-dicaféoylquinique.

2. Concentré de jus pressé de plante fraîche d'achillée millefeuille selon la revendication 1, dans lequel le concentré de jus pressé de plante fraîche d'achillée millefeuille comprend en outre des conservateurs et/ou des stabilisants.

3. Concentré de jus pressé de plante fraîche d'achillée millefeuille selon la revendication 1 ou 2, dans lequel le concentré de jus pressé de plante fraîche d'achillée millefeuille est produit ou peut être produit par un procédé qui comprend le séchage par pulvérisation.

4. Procédé de production d'un concentré de jus pressé de plante fraîche d'achillée millefeuille selon l'une quelconque des revendications précédentes, comprenant les étapes ou constitué des étapes consistant à:

fournir un jus pressé de plante fraîche d'achillée millefeuille, et

concentrer le jus pressé de plante fraîche d'achillée millefeuille.

5. Procédé selon la revendication 4, dans lequel le jus pressé de plante fraîche d'achillée millefeuille est concentré à une température < 60 °C.

6. Composition cosmétique comprenant du concentré de jus pressé de plante fraîche d'achillée millefeuille selon l'une quelconque des revendications 1 à 3.

7. Composition pharmaceutique, composition alimentaire ou complément alimentaire comprenant du concentré de jus pressé de plante fraîche d'achillée millefeuille selon l'une quelconque des revendications 1 bis 3.

8. Composition cosmétique selon la revendication 6 ou composition pharmaceutique selon la revendication 7, comprenant en outre entre 0,01 et 10 % en poids d'un agent apaisant pour la peau et/ou d'un antioxydant.

9. Utilisation cosmétique et non thérapeutique de l'achillée millefeuille pour stimuler l'expression des protéines de choc thermique (HSP) dans les cellules de la peau.

10. Utilisation cosmétique et non thérapeutique de l'achillée millefeuille pour augmenter la thermotolérance de la peau.

11. Utilisation de l'achillée millefeuille selon l'une quelconque des revendications 9 ou 10, dans laquelle l'achillée millefeuille est présente sous forme de jus pressé de plante fraîche d'achillée millefeuille ou sous forme de composition cosmétique selon l'une quelconque des revendications 6 ou 8.

12. Utilisation de l'achillée millefeuille selon la revendication 11, dans laquelle le dosage de concentré de jus pressé de plante fraîche d'achillée millefeuille dans la composition cosmétique est compris entre 0,0001 et 10 % en poids par rapport à 100 % en poids de la composition cosmétique.

13. Composition pharmaceutique selon la revendication 7, destinée à être utilisée en tant que médicament.

14. Utilisation de l'achillée millefeuille selon l'une quelconque des revendications 9 à 12, dans laquelle une application se fait par voie topique sur la peau et/ou les cheveux.

15. Composition pharmaceutique destinée à être utilisée en tant que médicament selon la revendication 13, dans laquelle une application se fait par voie topique sur la peau et/ou les cheveux.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9733596 A1
- WO 2006045743 A
- DE 29924656
- DE 69917811
- DE 69624799
- EP 2952103 A
- DE 69518581
- GB 1447988 A
- DE 19817177
- US 4960764 A, Figueroa
- US 4254105 A, Fukuda
- WO 2005123101 A
- DE 3740186
- DE 3938140
- DE 4204321
- DE 4229707
- DE 4309372
- DE 4411664
- DE 19541967
- DE 19543695
- DE 19543696
- DE 19547160
- DE 19602108
- DE 19602110
- DE 19602111
- DE 19631003
- DE 19631004
- DE 4229737
- DE 4237081
- DE 4324219
- DE 4429467
- DE 4423410
- DE 19516705
- DE 4333238 A
- DE 3929973
- DE 2150557
- DE 2817369
- DE 3708451
- US 9265743 B

**In der Beschreibung aufgeführte Nicht-Patentliteratur**

- *CHEMICAL ABSTRACTS,* 327-97-9
- *CHEMICAL ABSTRACTS,* 906-33-2
- *CHEMICAL ABSTRACTS,* 905-99-7
- *CHEMICAL ABSTRACTS,* 30964-13-7
- *CHEMICAL ABSTRACTS,* 14534-61-3
- *CHEMICAL ABSTRACTS,* 57378-72-0
- Sun Products Formulary. *Cosmetics & Toiletries,* Dezember 1990, vol. 105, 122-139
- Sun Products Formulary. *Cosmetics & Toiletries,* Marz 1987, vol. 102, 117-136
- Lexikon der Hilfsstoffe für Pharmazie. **FIEDLER, H. P.** Kosmetik und angrenzende Gebiete. ECV-Editio-Kantor-Verlag, 1996
- Hager's Handbuch der Pharmazeutischen Praxis. Springer Verlag
- **S. ARCTANDER.** Perfume and Flavor Chemicals. Eigenverlag, 1969
- **SURBURG ; PANTEN.** Common Fragrance and Flavor Materials. Wiley-VCH, 2006
- **SCHRADER ; GRUNDLAGEN ; REZEPTUREN.** Kosmetika. Hüthig Verlag, 1989
- **MAYTIN E. et al.** *J. Invest. Dermatol,* 1990, vol. 95, 635-42
- *J. Biol Chem.,* 1992, vol. 267 (231), 89-96
- *J. Invest Dermatol,* 1995, vol. 104 (4), 448-55
- **FARAGE MA.** Textbook of Aging Skin. Springer-Verlag, 2010